# EUROPEAN PATENT SPECIFICATION

(11) **EP 3 504 225 B1**
(45) Date of publication and mention of the grant of the patent: **24.04.2024**
(21) Application number: 18808514.6
(22) Date of filing: 16.11.2018
(51) Int. Cl.: C07K 14/155, A61K 39/21, C12N 15/49, C12N 5/10, G01N 33/543

(54) **NOVEL MAMMALIAN EXPRESSED HUMAN IMMUNODEFICIENCY VIRUS ENVELOPE PROTEIN ANTIGENS**
NEUE, IN SÄUGETIEREN EXPRIMIERTE HÜLLPROTEIN-ANTIGENE DES HUMANEN IMMUNSCHWÄCHEVIRUS
NOUVEAUX ANTIGÈNES PROTÉIQUES DE L'ENVELOPPE PROTÉIQUE DU VIRUS DE L'IMMUNODÉFICIENCE HUMAINE EXPRIMÉS CHEZ LES MAMMIFÈRES

(30) Priority: 17.11.2017 US 201762588085 P
(43) Date of publication of application: 03.07.2019
(60) Divisional application: 24164321.2
(73) Proprietor: Grifols Diagnostic Solutions Inc., Emeryville, CA 94608 (US)
(72) Inventor: MELTON WITT, Jody, Oakland, 94609 (US); SON, Sodany, Castro Valley, 94552 (US); BAUMEISTER, Mark, Willow Grove, Pennsylvania 19090 (US); BERRY, Jody, Easton, Pennsylvania 18040 (US)
(74) Representative: HGF
(86) International application number: PCT/IB2018/059037
(87) International publication number: WO 2019/097466

(56) References cited:
- EP-A1- 0 782 625
- WO-A1-2017/102929
- WO-A2-2004/009785
- WO-A2-2009/102357
- WO-A2-2009/137632
- US-B1- 6 284 248
- ZHANNA SHUBIN ET AL: "An HIV Envelope gp120-Fc Fusion Protein Elicits Effector Antibody Responses in Rhesus Macaques", CLINICAL AND VACCINE IMMUNOLOGY, vol. 24, no. 6, 12 April 2017 (2017-04-12) , XP055549292, US ISSN: 1556-6811, DOI: 10.1128/CVI.00028-17
- MARK MELCHERS ET AL: "A stabilized HIV-1 envelope glycoprotein trimer fused to CD40 ligand targets and activates dendritic cells", RETROVIROLOGY, vol. 8, no. 1, 1 January 2011 (2011-01-01) , pages 48-48, XP055010580, ISSN: 1742-4690, DOI: 10.1186/1742-4690-8-48
- QI Z ET AL: "A recombinant mimetics of the HIV-1 gp41 prehairpin fusion intermediate fused with human IgG Fc fragment elicits neutralizing antibody response in the vaccinated mice", BIOCHEMICAL AND BIOPHYSICAL RESEARCH COMMUNICATIONS, ELSEVIER, AMSTERDAM, NL, vol. 398, no. 3, 30 July 2010 (2010-07-30) , pages 506-512, XP027185030, ISSN: 0006-291X, DOI: 10.1016/J.BBRC.2010.06.109 [retrieved on 2010-07-01]
- ZHANG M Y ET AL: "Cross-reactive HIV-1-neutralizing activity of serum IgG from a rabbit immunized with gp41 fused to IgG1 Fc: Possible role of the prolonged half-life of the immunogen", VACCINE, ELSEVIER, AMSTERDAM, NL, vol. 27, no. 6, 5 February 2009 (2009-02-05), pages 857-863, XP026139089, ISSN: 0264-410X, DOI: 10.1016/J.VACCINE.2008.11.083 [retrieved on 2009-02-05]

## Description

The present application is being filed along with an electronic Sequence Listing as an ASCII text file via EFS-Web. The electronic Sequence Listing is provided as a file entitled SEQLIST1800393.txt, created and last saved on Nov 15, 2018, which is 501,813 bytes in size. The information in the electronic Sequence Listing is incorporated herein by reference in its entirety.

### FIELD

The present disclosure is related to the field of recombinant proteins. Some embodiments of the present disclosure relate to HIV envelope recombinant polypeptides comprising at least one epitope of the HIV gp120 and gp41 proteins and a fusion protein. The present disclosure is also related to methods and devices for assaying a sample containing HIV antibodies.

### BACKGROUND

HIV caused a global epidemic leading to the death of an estimated 35 million people due to AIDS-related illnesses. An estimated 1.8 million new cases of HIV occurred in 2016 and roughly 36.7 million people around the world are infected. HIV is a member of the retrovirus family in the lentivirus subgroup and has two described types: HIV-1 and HIV-2. HIV-1 is more infectious and virulent than HIV-2, and it causes the majority of HIV infections worldwide.

Each HIV virion contains two copies of RNA that encode nine genes and nineteen proteins. The sole proteins on the surface of an intact virion are the envelope glycoproteins encoded by the env gene. The product of the *env* gene is an approximately 850 amino acid precursor protein called gp160. This protein is cleaved by the cellular protease furin in the endoplasmic reticulum into two cleavage products gp120 and gp41. Both gp120 and gp41 exist as trimers on the cell surface to form a heterodimer 'spike'. Gp41 is a transmembrane glycoprotein that acts as an anchor for the extracellular gp120, to which it is non-covalently bound. Gp120 contains binding sites for cellular receptors on lymphocytes and is critical for viral entry into cells.

As the sole proteins on the virion surface, gp120 and gp41 expose a number of immunodominant epitopes targeted by the host immune system. Versions of these proteins have been utilized historically in immunoassays to capture and detect antibodies mounted against them as confirmation of HIV infection.

Gp120 and gp41 are useful in vaccinations, in diagnostic assays, and in the development of diagnostic assays. Recombinant gp120 and gp41 are typically produced in yeast, but the production of gp120 and gp41 in yeast is challenging because the proteins may misfold, aggregate, and/or accrue aberrant post-translational modifications. Additionally, the purification of gp120 and gp41 from yeast presents an arduous multi-step process. Improved compositions and methods for producing gp120 and gp41 antigens are therefore desirable.

### SUMMARY

The invention is defined by the appended claims.

The invention relates to a recombinant monomeric polypeptide, comprising a leader peptide, and a fusion protein, comprising at least one epitope of the HIV gp120 protein, and at least one epitope of the HIV gp41 protein; wherein the fusion protein comprises a region that is not encoded by HIV, said region selected from the group consisting of a Fc domain of an antibody that lacks a hinge region, and wherein the recombinant polypeptide lacks a transmembrane domain, and wherein the polypeptide include at least 4 epitopes of the HIV envelope protein.

A recombinant polypeptide according to the present invention lacks a transmembrane domain, said transmembrane domain having an amino acid sequence selected from the group consisting of SEQ ID NO:63, SEQ ID NO:64, SEQ ID NO:65, SEQ ID NO:66, SEQ ID NO:67, SEQ ID NO:68, SEQ ID NO:69, SEQ ID NO:70, SEQ ID NO:71, or SEQ ID NO:72.

A recombinant polypeptide according to the present invention may comprise a plurality of epitopes of the HIV envelope protein. In some embodiments, the recombinant polypeptide comprises at least 4, 5, 6, 7, 8, 9, or 10 epitopes. In some embodiments each epitope is connected to another epitope by a linker, which amino acid sequence is not encoded by HIV. In some embodiments, the linker connecting each epitope to another epitope comprises the amino acid sequence set forth in SEQ ID NO: 73, SEQ ID NO:74, SEQ ID NO:75, SEQ ID NO:76, SEQ ID NO:109, SEQ ID NO:110 or SEQ ID NO:111.

A polypeptide may comprise a plurality of epitopes of the HIV envelope protein, wherein each epitope of the plurality of epitopes is connected to another epitope of the plurality of epitopes by an amino acid sequence that is not encoded by HIV. For example, each epitope of the plurality of epitopes may be connected to another epitope of the plurality of epitopes by a linker. In some embodiments, each epitope of the plurality of epitopes may be connected to another epitope of the plurality of epitopes by a linker, *e.g.,* wherein each linker comprises an alpha helix or a beta strand. A plurality of epitopes may comprise at least 2, 3, 4, 5, 6, 7, 8, 9, or 10 epitopes. A polypeptide comprising a plurality of epitopes may comprise the amino acid sequence set forth in SEQ ID NO:1, SEQ ID NO:2, SEQ ID NO:3, SEQ ID NO:4, SEQ ID NO:5, SEQ ID NO:6, SEQ ID NO:7, SEQ ID NO:8, SEQ ID NO:20, SEQ ID NO:21, or SEQ ID NO:22, or an amino acid sequence having at least about 80%, 85%, 90%, 95%, 96%, 97%, 98%, or 99% sequence identity with the amino acid sequence set forth in SEQ ID NO:1, SEQ ID NO:2, SEQ ID NO:3, SEQ ID NO:4, SEQ ID NO:5, SEQ ID NO:6, SEQ ID NO:7, SEQ ID NO:8, SEQ ID NO:20, SEQ ID NO:21, or SEQ ID NO:22.

A polypeptide may comprise one or more epitopes of the HIV gp120 protein, and one or more epitopes of the HIV gp41 protein.

In some embodiments, the recombinant polypeptide comprises the amino acid sequence set forth in SEQ ID NO:14, SEQ ID NO:16, SEQ ID NO:21, SEQ ID NO:22, SEQ ID NO:23, SEQ ID NO:24, SEQ ID NO:25, SEQ ID NO:26, SEQ ID NO:27, SEQ ID NO:28, SEQ ID NO:29, SEQ ID NO:30, SEQ ID NO:31, SEQ ID NO:91, SEQ ID NO:92, SEQ ID NO:93, SEQ ID NO:94, SEQ ID NO:95, SEQ ID NO:96, SEQ ID NO:97, SEQ ID NO:98, SEQ ID NO:99, SEQ ID NO:100,SEQ ID NO:101, SEQ ID NO:102, SEQ ID NO:103, SEQ ID NO:104, SEQ ID NO:105, or SEQ ID NO:106, or an amino acid sequence having at least about 80%, 85%, 90%, 95%, 96%, 97%, 98%, or 99% sequence identity with the amino acid sequence set forth in SEQ ID NO:1, SEQ ID NO:2, SEQ ID NO:3, SEQ ID NO:4, SEQ ID NO:5, SEQ ID NO:6, SEQ ID NO:7, SEQ ID NO:8, SEQ ID NO:9, SEQ ID NO:14, SEQ ID NO:15, SEQ ID NO:16, SEQ ID NO:17, SEQ ID NO:18, SEQ ID NO:19, SEQ ID NO:20, SEQ ID NO:21, SEQ ID NO:22, SEQ ID NO:23, SEQ ID NO:24, SEQ ID NO:25, SEQ ID NO:26, SEQ ID NO:27, SEQ ID NO:28, SEQ ID NO:29, SEQ ID NO:30, SEQ ID NO:31, SEQ ID NO:32, SEQ ID NO:33, SEQ ID NO:91, SEQ ID NO:92, SEQ ID NO:93, SEQ ID NO:94, SEQ ID NO:95, SEQ ID NO:96, SEQ ID NO:97, SEQ ID NO:98, SEQ ID NO:99, SEQ ID NO:100,SEQ ID NO:101, SEQ ID NO:102, SEQ ID NO:103, SEQ ID NO:104, SEQ ID NO:105, or SEQ ID NO:106.

In all the embodiments, the fusion protein of the recombinant polypeptide comprise an antibody Fc domain. In some embodiments the fusion protein comprises an antibody Fc domain which amino acid sequence is selected from the group consisting of SEQ ID. NO: 77, or SEQ ID. NO: 80.

In all the embodiments, the antibody Fc domain of the fusion protein lacks an oligomerization domain, and the recombinant polypeptide is a monomer.

The polypeptide comprise an epitope of HIV gp120 and HIV gp41. A polypeptide may comprise 1, 2, 3, 4, 5, 6, 7, 8, 9, or 10 epitopes of HIV gp120 and 1, 2, 3, 4, 5, 6, 7, 8, 9, or 10 epitopes of HIV gp41.

The polypeptide comprises a leader peptide. In some embodiments the N-terminus of a recombinant polypeptide may consist of a leader peptide and the leader peptide is capable of translocating the recombinant polypeptide outside of the cell surface membrane of a mammalian cell (*e.g*., a human cell) following the translation of the polypeptide in the mammalian cell. In some embodiments the leader peptide comprises the amino acid sequence set forth in SEQ ID NO:84.

In some embodiments, the fusion protein of the recombinant polypeptide is the leader peptide, although the fusion protein is typically not the leader peptide. In embodiments wherein the fusion protein is the leader peptide, the recombinant polypeptide comprises an epitope of HIV gp120 and an epitope of HIV gp41, and the recombinant polypeptide lacks the gp120/gp41 cleavage site. Such polypeptides may comprise the amino acid sequence set forth in SEQ ID NO:34, SEQ ID NO:35, SEQ ID NO:36, SEQ ID NO:37, or SEQ ID NO:38 or an amino acid sequence having at least about 80%, 85%, 90%, 95%, 96%, 97%, 98%, or 99% sequence identity with the amino acid sequence set forth in SEQ ID NO:34, SEQ ID NO:35, SEQ ID NO:36, SEQ ID NO:37, or SEQ ID NO:38.

In some embodiments, the recombinant polypeptide further comprises a polyhistidine affinity tag.

Disclosed is also a cell comprising a polypeptide as described herein, wherein the cell is a mammalian cell such as a human cell. The cell may be, for example, a CHO, BHK, NS0, Sp2/0, COS, C127, HEK293, HT-1080, PER.C6, HeLa, or Jurkat cell.

Disclosed is also a nucleic acid comprising a nucleotide sequence encoding the polypeptide of any one of the embodiments and a promoter operably linked to the nucleotide sequence. The nucleotide sequence may be codon optimized for expression in mammalian cells (such as human cells). The promoter may be capable of driving expression in mammalian cells (such as human cells). The disclosure also relates to a cell comprising a nucleic acid comprising a nucleotide sequence that encodes a polypeptide described herein. The cell may be a cloning cell (*e.g*., *E. coli*) or an expression cell (*e.g.*, a CHO, BHK, NS0, Sp2/0, COS, C127, HEK293, HT-1080, PER.C6, HeLa, or Jurkat cell).

Some embodiments of the invention relate to a device for assaying a composition containing an anti-HIV antibody, comprising a solid support and a polypeptide as described herein, wherein the polypeptide is covalently or non-covalently bound to the solid support. The composition may comprise a bodily fluid such as blood, blood plasma, blood serum, or saliva, *e.g.,* obtained from a human patient. The solid support may be a bead, a membrane, a microtiter plate, a polypeptide chip, or the solid-phase of a chromatography column.

Some embodiments of the invention relate to an immunoassay reagent comprising a polypeptdide as described herein, wherein the immunoassay reagent is bound to a solid support. In some embodiments, the solid support is a bead, a membrane, a microtiter plate, a polypeptide chip, or the solid-phase of a chromatography column.

Some embodiments of the invention relate to a method of assaying a sample containing an antibody, comprising contacting the sample and a polypeptide as described herein and determining the binding affinity between the antibody and the polypeptide. In some embodiments the binding affinity may be, a relative binding affinity, wherein the binding affinity is a relative binding affinity because it is relative to the binding affinity of one or more different antibodies.

Some embodiments of the invention relate to a method of selecting an anti-HIV antibody from a plurality of antibodies, comprising contacting a polypeptide as described herein and a composition containing the plurality of antibodies and identifying an antibody that has a high binding affinity to the polypeptide relative to other antibodies in the composition, thereby selecting the anti-HIV antibody.

Some embodiments of the invention relate to the recombinant monomeric polypeptide for use in preventing or treating an HIV infection in a human patient, comprising administering to the patient a recombinant polypeptide as described herein.

Disclosed is also a method of selecting biological samples from a supply of human biological samples, comprising selecting from said supply those samples that do not comprise antibodies that form an antigen-antibody complex with a recombinant polypeptide as described herewith.

### BRIEF DESCRIPTION OF THE DRAWINGS

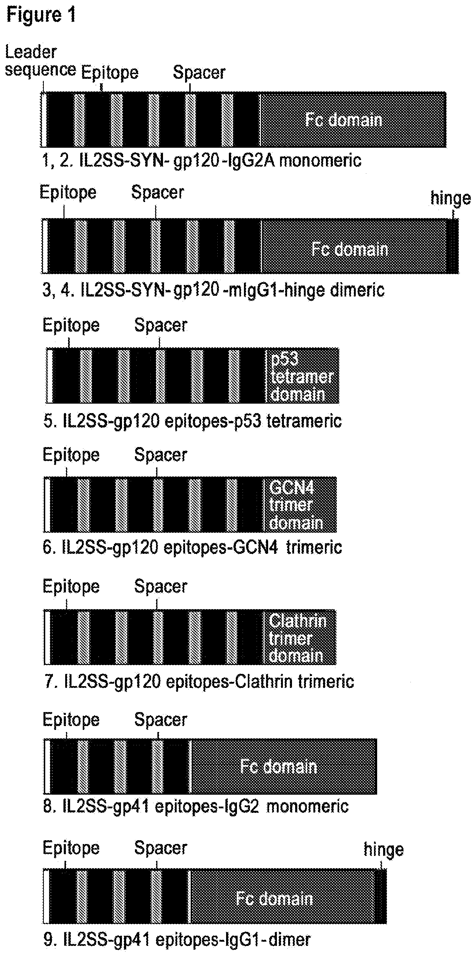

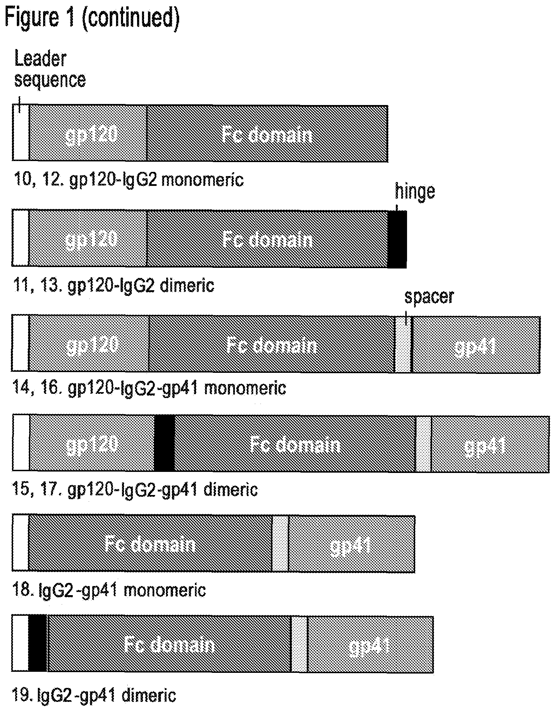

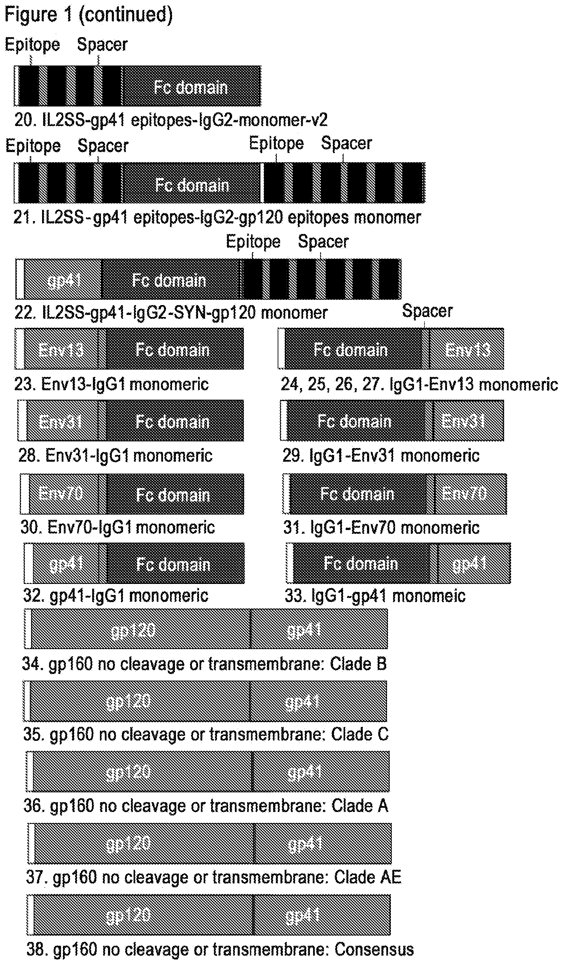

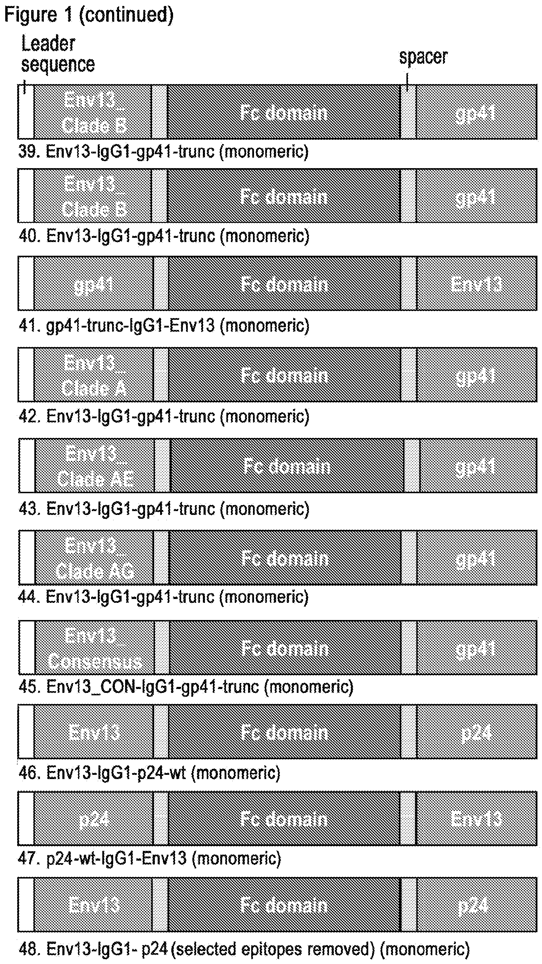

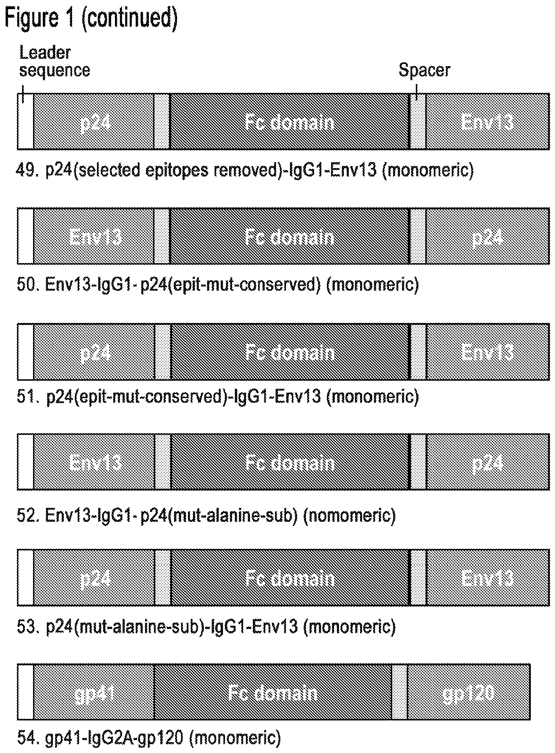

Figure 1 contains schematic diagrams of polypeptides env_1 to env_38, env_39 to env_42 and env_43 to env_54, which are described in the exemplification section and have the amino acid sequences set forth in SEQ ID NO:1-38, SEQ ID NO:92 to 94 and SEQ ID NO:95 to 106, respectively.

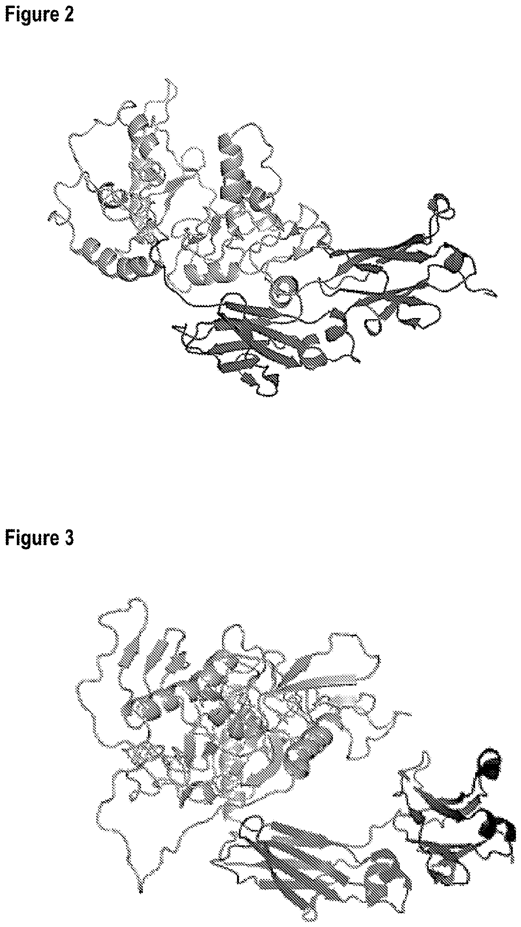

Figure 2 is an image of the predicted structure of a recombinant polypeptide having the amino acid sequence set forth in SEQ ID NO:1, wherein the dark portion of the structure comprises a fusion protein as an antibody Fc domain, and the light portion comprises a plurality of epitopes of HIV gp120.
Figure 3 is an image of the predicted structure of a recombinant polypeptide having the amino acid sequence set forth in SEQ ID NO:2, wherein the dark portion of the structure comprises a fusion protein as an antibody Fc domain, and the light portion comprises a plurality of epitopes of HIV gp120.

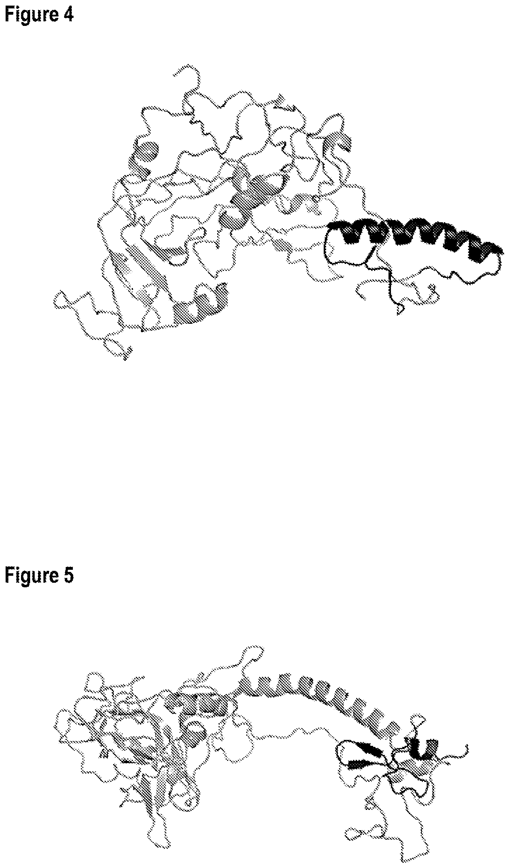

Figure 4 is an image of the predicted structure of a recombinant polypeptide having the amino acid sequence set forth in SEQ ID NO:5, wherein the dark portion of the structure comprises a fusion protein as a p53 tetramerization domain, and the light portion comprises a plurality of epitopes of HIV gp120.
Figure 5 is an image of the predicted structure of a recombinant polypeptide having the amino acid sequence set forth in SEQ ID NO:6, comprising a GCN4 trimerization domain and a plurality of epitopes of HIV gp120.

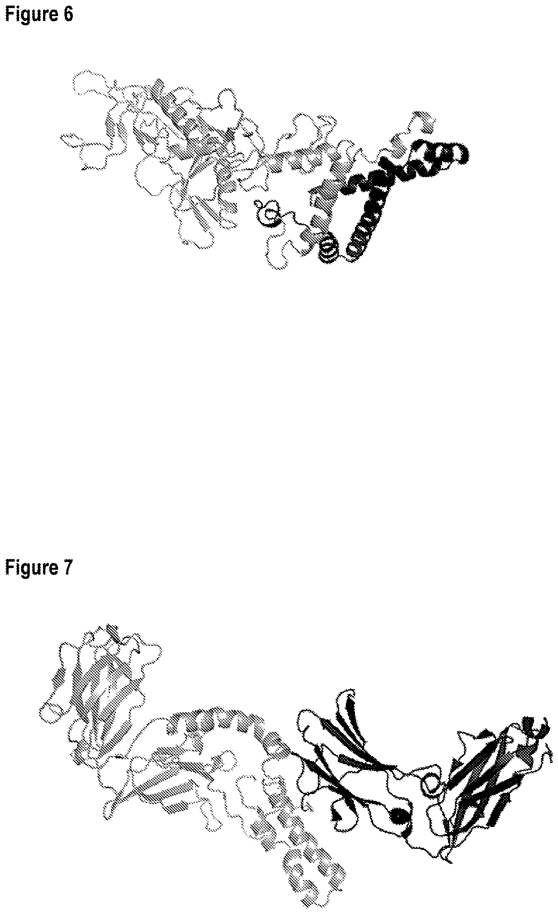

Figure 6 is an image of the predicted structure of a recombinant polypeptide having the amino acid sequence set forth in SEQ ID NO:7, wherein the dark portion of the structure comprises a fusion protein as a clathrin trimerization domain, and the light portion comprises a plurality of epitopes of HIV gp120.
Figure 7 is an image of the predicted structure of a recombinant polypeptide having the amino acid sequence set forth in SEQ ID NO:8, wherein the dark portion of the structure comprises a fusion protein as an antibody Fc domain, and the light portion comprises a plurality of epitopes of HIV gp41.

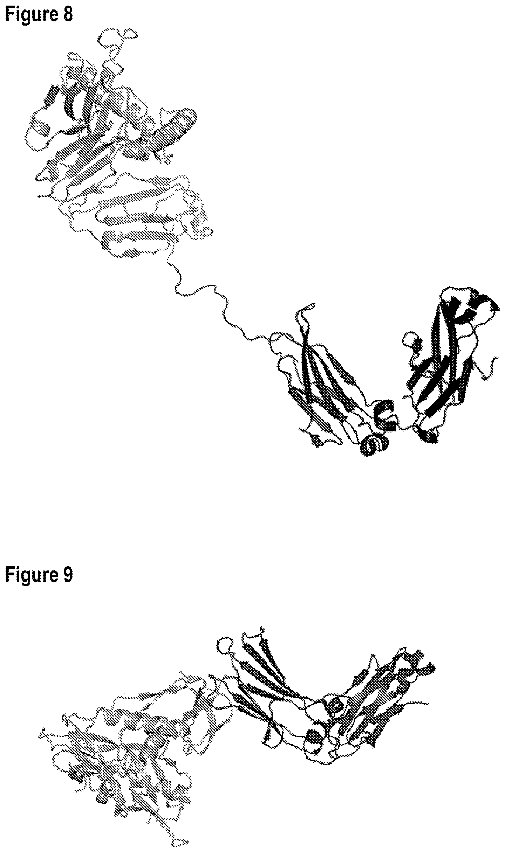

Figure 8 is an image of the predicted structure of a recombinant polypeptide having the amino acid sequence set forth in SEQ ID NO:9, wherein the dark portion of the structure comprises a fusion protein as an antibody Fc domain, and the light portion comprises a plurality of epitopes of HIV gp41.
Figure 9 is an image of the predicted structure of a recombinant polypeptide having the amino acid sequence set forth in SEQ ID NO:10, wherein the dark portion of the structure comprises a fusion protein as an antibody Fc domain, and the light portion comprises HIV gp120 epitopes.

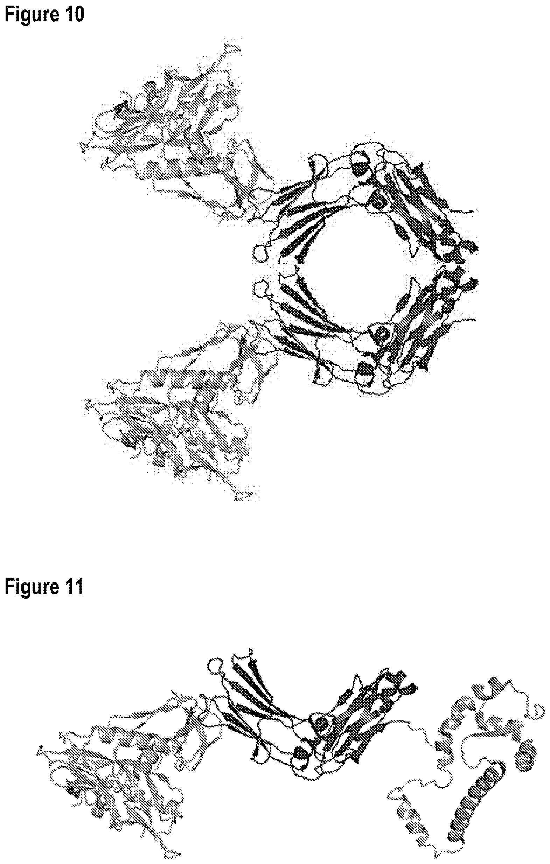

Figure 10 is an image of the predicted structure of a recombinant polypeptide dimer having the amino acid sequence set forth in SEQ ID NO:13, wherein the dark portion of the structure comprises fusion proteins as antibody Fc domains, and the light portions comprise HIV gp120 epitopes.
Figure 11 is an image of the predicted structure of a recombinant polypeptide having the amino acid sequence set forth in SEQ ID NO:14, wherein the dark portion of the structure comprises a fusion protein as an antibody Fc domain, and the light portions comprise HIV gp120 epitopes (left) and HIV gp41 epitopes (right).

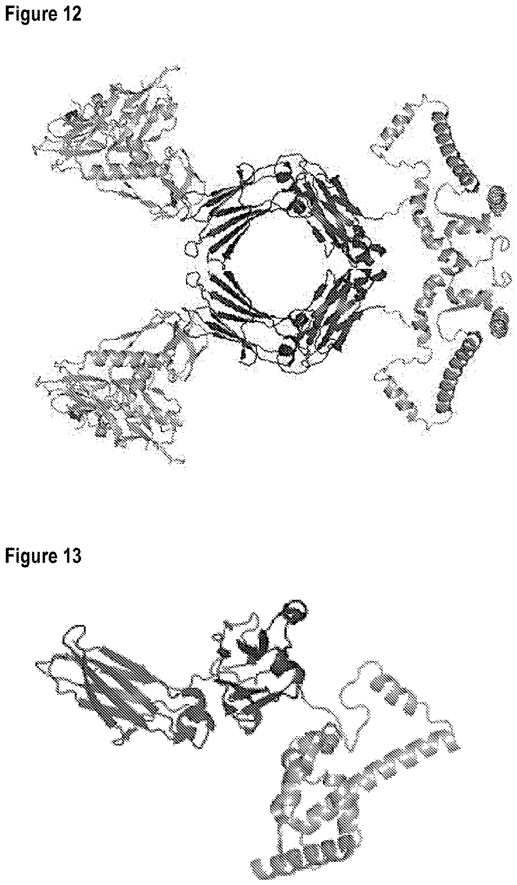

Figure 12 is an image of the predicted structure of a recombinant polypeptide dimer having the amino acid sequence set forth in SEQ ID NO:15, wherein the dark portion of the structure comprises fusion proteins as antibody Fc domains, and the light portions comprise HIV gp120 epitopes (left) and HIV gp41 epitopes (right).
Figure 13 is an image of the predicted structure of a recombinant polypeptide having the amino acid sequence set forth in SEQ ID NO:18, wherein the dark portion of the structure comprises a fusion protein as an antibody Fc domain, and the light portion comprises HIV gp41 epitopes.

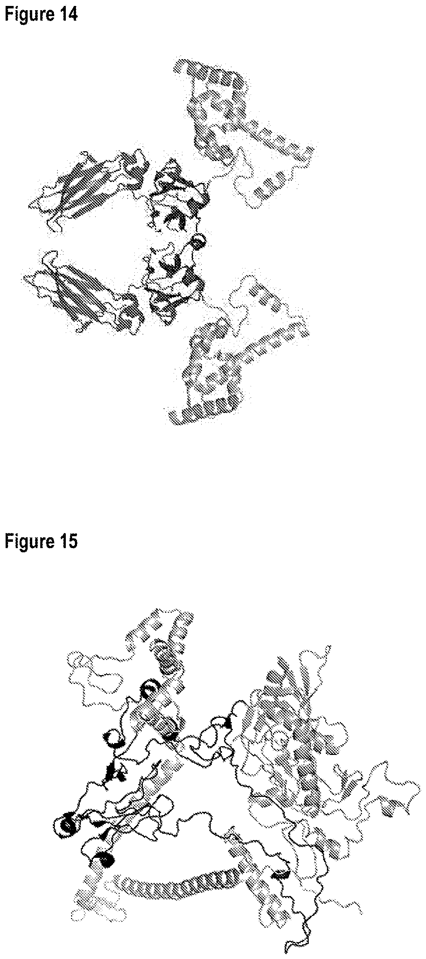

Figure 14 is an image of the predicted structure of a recombinant polypeptide dimer having the amino acid sequence set forth in SEQ ID NO:19, wherein the dark portion of the structure comprises fusion proteins as antibody Fc domains, and the light portions comprise HIV gp41 epitopes.
Figure 15 is an image of the predicted structure of a recombinant polypeptide having the amino acid sequence set forth in SEQ ID NO:21 , wherein the dark portion of the structure comprises a fusion protein as an antibody Fc domain, and the light portions comprise a plurality of epitopes of HIV gp41 and a plurality of epitopes of HIV gp120.

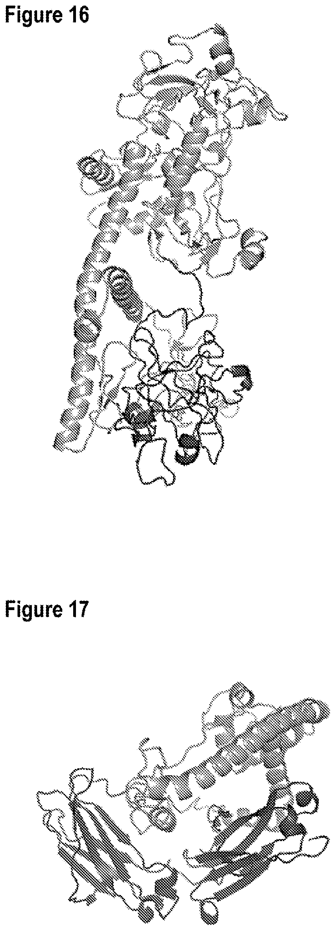

Figure 16 is an image of the predicted structure of a recombinant polypeptide having the amino acid sequence set forth in SEQ ID NO:22, wherein the dark portion of the structure comprises a fusion protein as an antibody Fc domain, and the light portions comprise HIV gp120 epitopes and a plurality of epitopes of HIV gp41.
Figure 17 is an image of the predicted structure of a recombinant polypeptide having the amino acid sequence set forth in SEQ ID NO:23, wherein the dark portion of the structure comprises a fusion protein as an antibody Fc domain, and the light portion comprises HIV envelope protein epitopes.

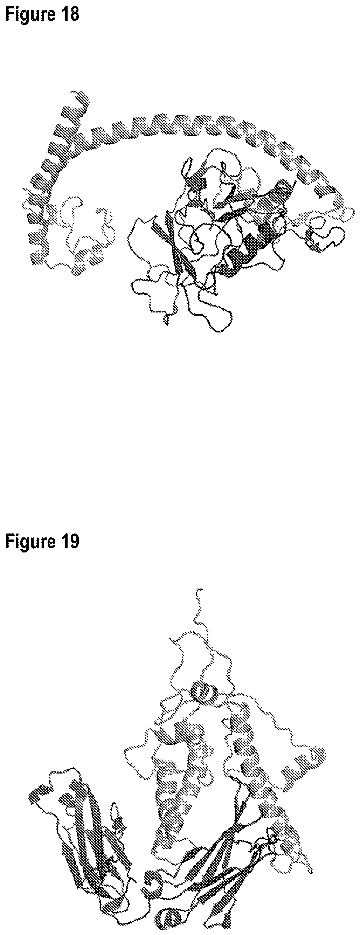

Figure 18 is an image of the predicted structure of a recombinant polypeptide having the amino acid sequence set forth in SEQ ID NO:24, wherein the dark portion of the structure comprises a fusion protein as an antibody Fc domain, and the light portion comprises HIV envelope protein epitopes.
Figure 19 is an image of the predicted structure of a recombinant polypeptide having the amino acid sequence set forth in SEQ ID NO:28, wherein the dark portion of the structure comprises a fusion protein as an antibody Fc domain, and the light portion comprises HIV envelope protein epitopes.

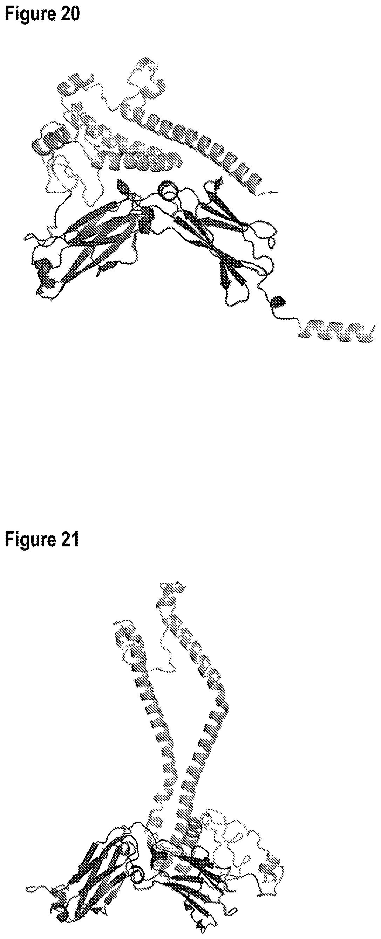

Figure 20 is an image of the predicted structure of a recombinant polypeptide having the amino acid sequence set forth in SEQ ID NO:29, wherein the dark portion of the structure comprises a fusion protein as an antibody Fc domain, and the light portion comprises HIV envelope protein epitopes.
Figure 21 is an image of the predicted structure of a recombinant polypeptide having the amino acid sequence set forth in SEQ ID NO:30, wherein the dark portion of the structure comprises a fusion protein as an antibody Fc domain, and the light portion comprises HIV envelope protein epitopes.

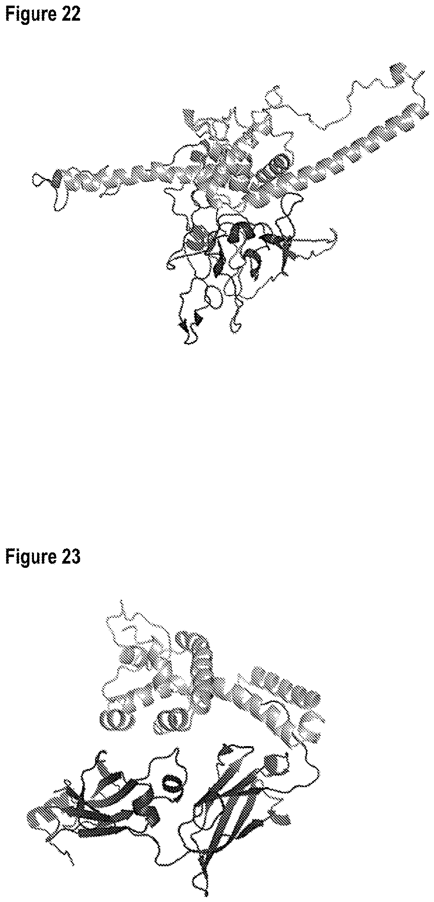

Figure 22 is an image of the predicted structure of a recombinant polypeptide having the amino acid sequence set forth in SEQ ID NO:31, wherein the dark portion of the structure comprises a fusion protein as an antibody Fc domain, and the light portion comprises HIV envelope protein epitopes.
Figure 23 is an image of the predicted structure of a recombinant polypeptide having the amino acid sequence set forth in SEQ ID NO:32, wherein the dark portion of the structure comprises gp41 epitopes.

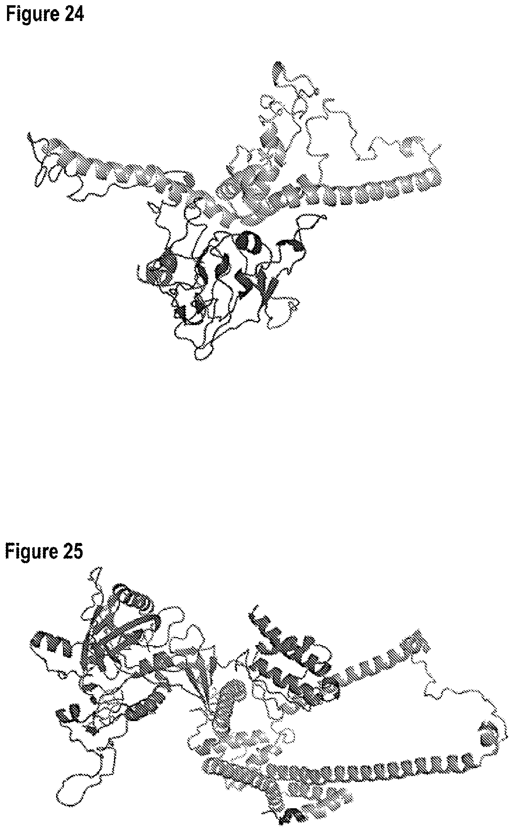

Figure 24 is an image of the predicted structure of a recombinant polypeptide having the amino acid sequence set forth in SEQ ID NO:33, wherein the dark portion of the structure comprises a fusion protein as an antibody Fc domain, and the light portion comprises HIV gp41 epitopes.
Figure 25 is an image of the predicted structure of a recombinant polypeptide having the amino acid sequence set forth in SEQ ID NO:34, comprising HIV gp41 epitopes and HIV gp120 epitopes.

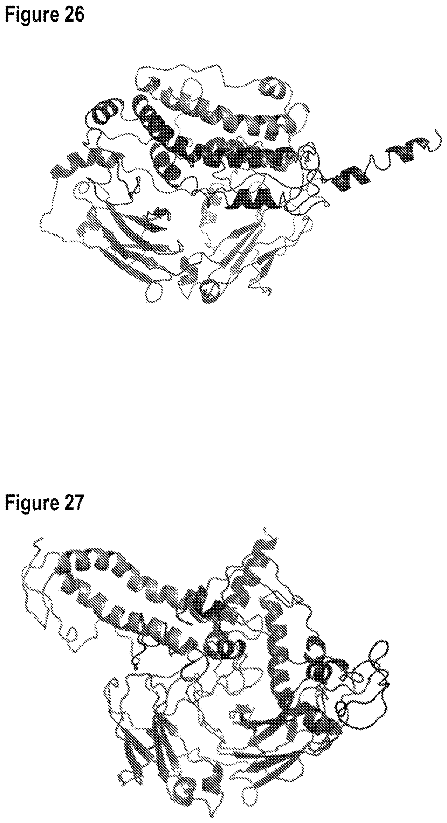

Figure 26 is an image of the predicted structure of a recombinant polypeptide having the amino acid sequence set forth in SEQ ID NO: 92, wherein the light grey portion comprises the HIV gp120 epitopes, the medium grey comprises the antibody Fc domain, the dark grey comprises the HIV gp41 epitopes, and the black comprises the linkers.
Figure 27 is an image of the predicted structure of a recombinant polypeptide having the amino acid sequence set forth in SEQ ID NO: 93, wherein the light grey portion comprises the HIV gp41 epitopes, the medium grey comprises the antibody Fc domain, the dark grey comprises the HIV g120 epitopes, and the black comprises the linkers.

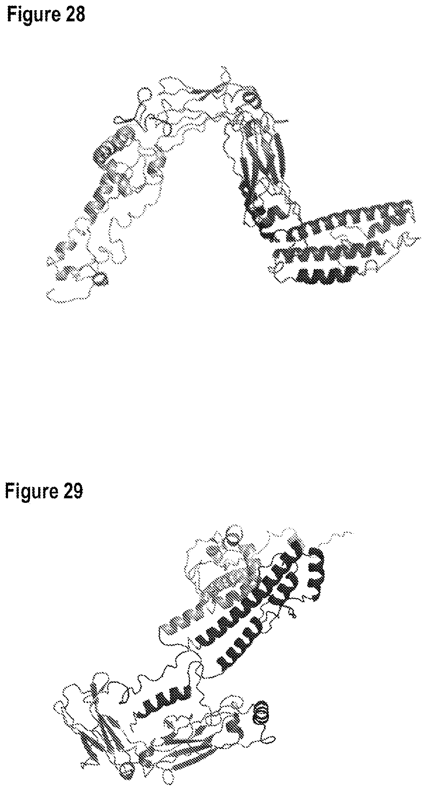

Figure 28 is an image of the predicted structure of a recombinant polypeptide having the amino acid sequence set forth in SEQ ID NO: 94, wherein the light grey portion comprises the HIV gp120 epitopes, the medium grey comprises the antibody Fc domain, the dark grey comprises the HIV g41 epitopes, and the black comprises the linkers.
Figure 29 is an image of the predicted structure of a recombinant polypeptide having the amino acid sequence set forth in SEQ ID NO: 95, wherein the light grey portion comprises the HIV gp120 epitopes, the medium grey comprises the antibody Fc domain, the dark grey comprises the HIV g41 epitopes, and the black comprises the linkers.

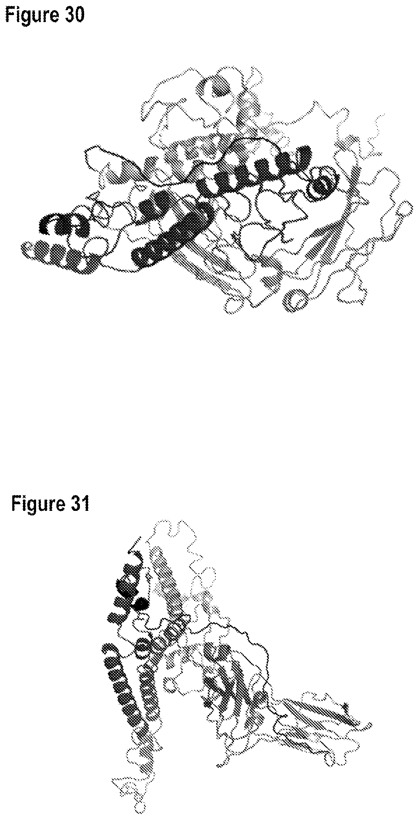

Figure 30 is an image of the predicted structure of a recombinant polypeptide having the amino acid sequence set forth in SEQ ID NO: 96, wherein the light grey portion comprises the HIV gp120 epitopes, the medium grey comprises the antibody Fc domain, the dark grey comprises the HIV g41 epitopes, and the black comprises the linkers.
Figure 31 is an image of the predicted structure of a recombinant polypeptide having the amino acid sequence set forth in SEQ ID NO: 97, wherein the light grey portion comprises the HIV gp120 epitopes, the medium grey comprises the antibody Fc domain, the dark grey comprises the HIV g41 epitopes, and the black comprises the linkers.

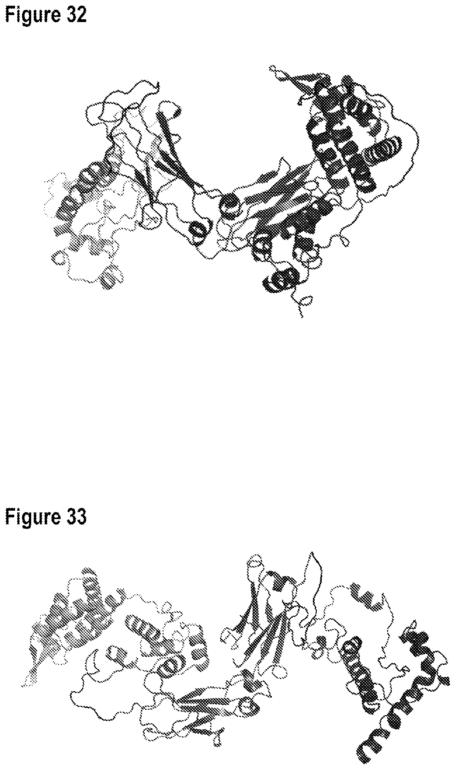

Figure 32 is an image of the predicted structure of a recombinant polypeptide having the amino acid sequence set forth in SEQ ID NO: 98, wherein the light grey portion comprises the HIV gp120 epitopes, the medium grey comprises the antibody Fc domain, the dark grey comprises the HIV p24 epitopes, and the black comprises the linkers.
Figure 33 is an image of the predicted structure of a recombinant polypeptide having the amino acid sequence set forth in SEQ ID NO: 99, wherein the light grey portion comprises the HIV p24 epitopes, the medium grey comprises the antibody Fc domain, the dark grey comprises the HIV gp120 epitopes, and the black comprises the linkers.

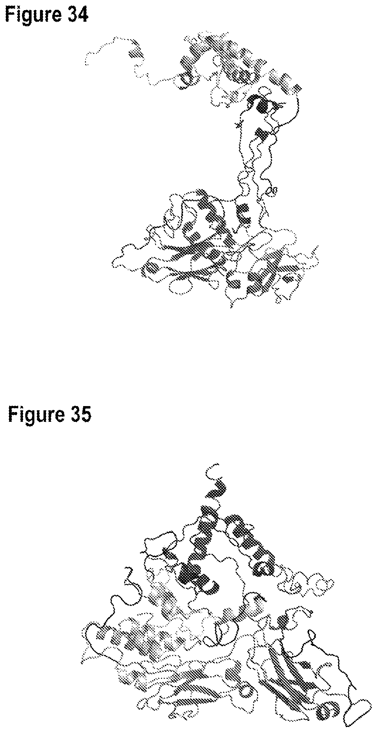

Figure 34 is an image of the predicted structure of a recombinant polypeptide having the amino acid sequence set forth in SEQ ID NO: 100, wherein the light grey portion comprises the HIV gp120 epitopes, the medium grey comprises the antibody Fc domain, the dark grey comprises the HIV p24 epitopes, and the black comprises the linkers.
Figure 35 is an image of the predicted structure of a recombinant polypeptide having the amino acid sequence set forth in SEQ ID NO: 101, wherein the light grey portion comprises the HIV p24 epitopes, the medium grey comprises the antibody Fc domain, the dark grey comprises the HIV gp120 epitopes, and the black comprises the linkers.

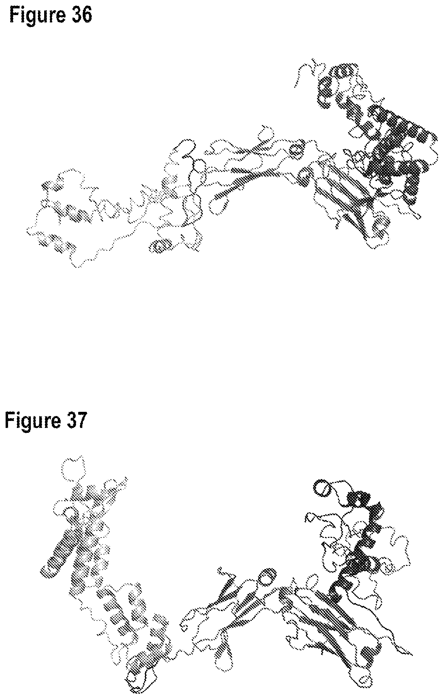

Figure 36 is an image of the predicted structure of a recombinant polypeptide having the amino acid sequence set forth in SEQ ID NO: 102, wherein the light grey portion comprises the HIV gp120 epitopes, the medium grey comprises the antibody Fc domain, the dark grey comprises the HIV p24 epitopes, and the black comprises the linkers.
Figure 37 is an image of the predicted structure of a recombinant polypeptide having the amino acid sequence set forth in SEQ ID NO: 103, wherein the light grey portion comprises the HIV p24 epitopes, the medium grey comprises the antibody Fc domain, the dark grey comprises the HIV gp120 epitopes, and the black comprises the linkers.

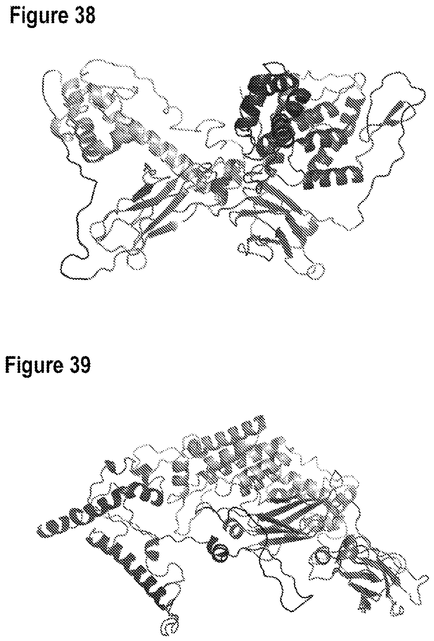

Figure 38 is an image of the predicted structure of a recombinant polypeptide having the amino acid sequence set forth in SEQ ID NO: 104, wherein the light grey portion comprises the HIV gp120 epitopes, the medium grey comprises the antibody Fc domain, the dark grey comprises the HIV p24 epitopes, and the black comprises the linkers.
Figure 39 is an image of the predicted structure of a recombinant polypeptide having the amino acid sequence set forth in SEQ ID NO: 105, wherein the light grey portion comprises the HIV p24 epitopes, the medium grey comprises the antibody Fc domain, the dark grey comprises the HIV gp120 epitopes, and the black comprises the linkers.

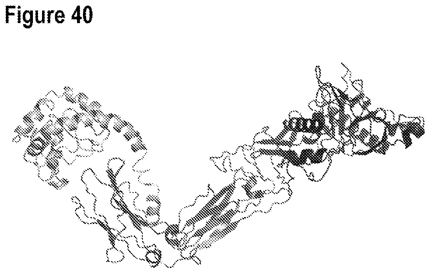

Figure 40 is an image of the predicted structure of a recombinant polypeptide having the amino acid sequence set forth in SEQ ID NO: 106, wherein the light grey portion comprises the HIV gp41 epitopes, the medium grey comprises the antibody Fc domain, the dark grey comprises the HIV gp120 epitopes, and the black comprises the linkers.

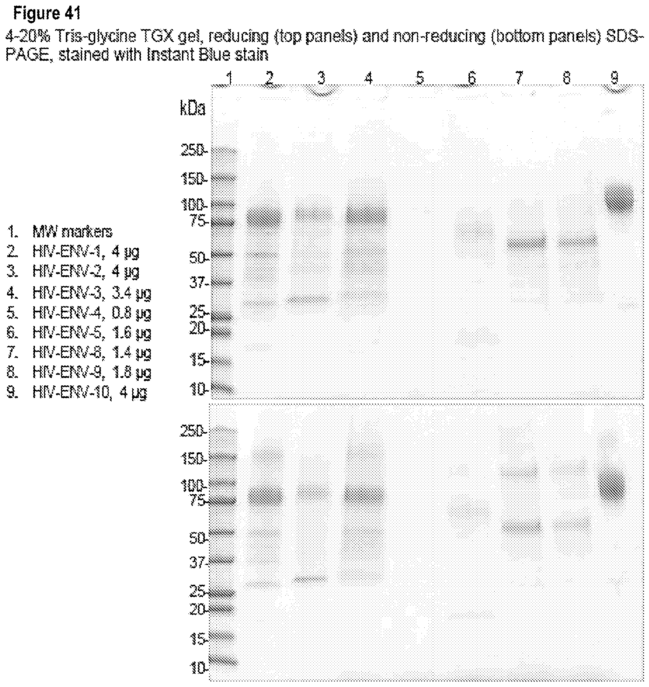

Figure 41 is an image of 4-20% tris-glycine TGX^{™} sodium dodecyl sulfate polyacrylamide (SDS-PAGE) gels run under reducing (top image) and non-reducing (bottom image) conditions. The gels were stained with the InstantBlue^{™} protein stain. The lanes of each gel are numbered 1 to 9 from left to right. The first lane contains a molecular weight marker, and lanes 2-9 contain polypeptides env_1, 2, 3, 4, 5, 8, 9, and 10, which were cloned and then expressed in eukaryotic cells. Polypeptides env_1, 2, 3, 5, 8, 9, and 10 expressed at detectable levels.

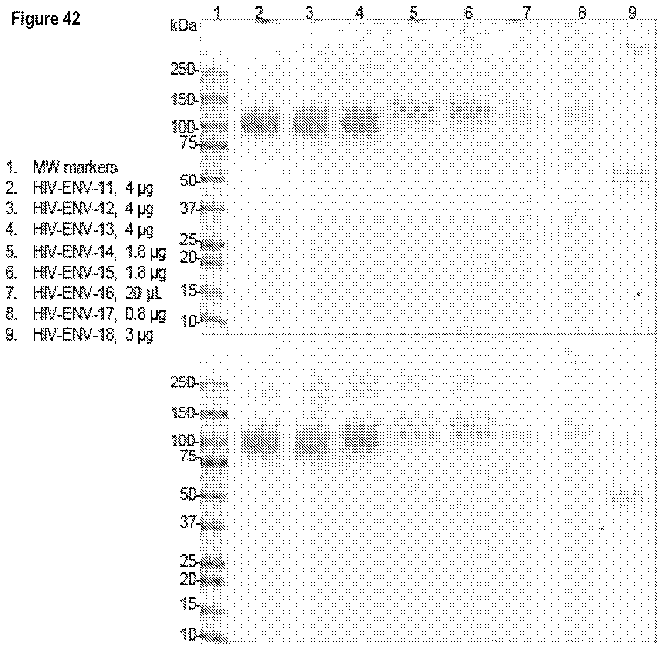

Figure 42 is an image of 4-20% tris-glycine TGX^{™} SDS-PAGE gels run under reducing (top image) and non-reducing (bottom image) conditions. The gels were stained with the InstantBlue^{™} protein stain. The lanes of each gel are numbered 1 to 9 from left to right. The first lane contains a molecular weight marker, and lanes 2-9 contain polypeptides env_11, 12, 13, 14, 15, 16, 17, and 18, which were cloned and then expressed in eukaryotic cells. Each polypeptide was capable of expression at a detectable level.

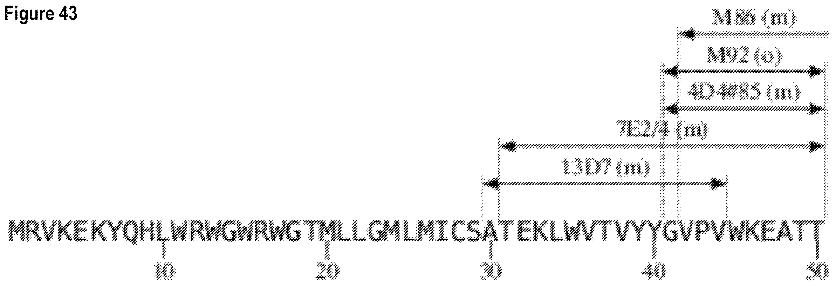

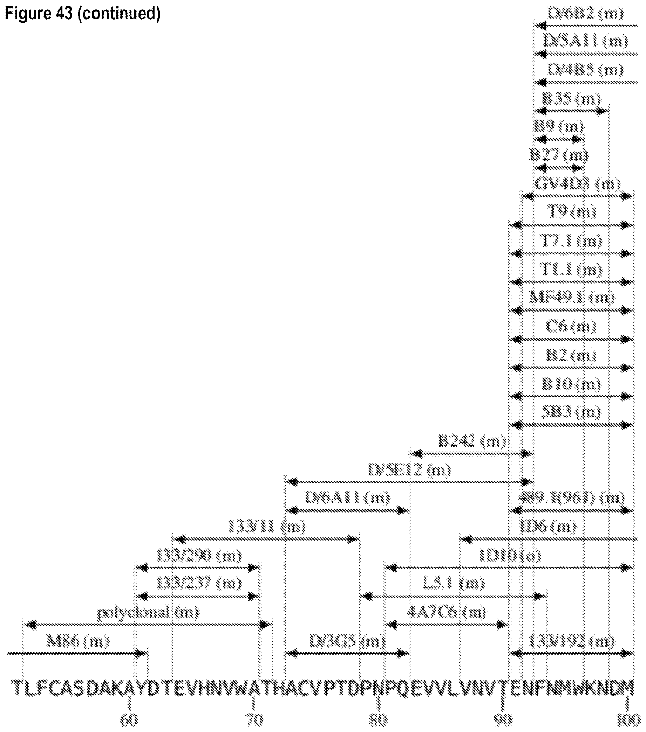

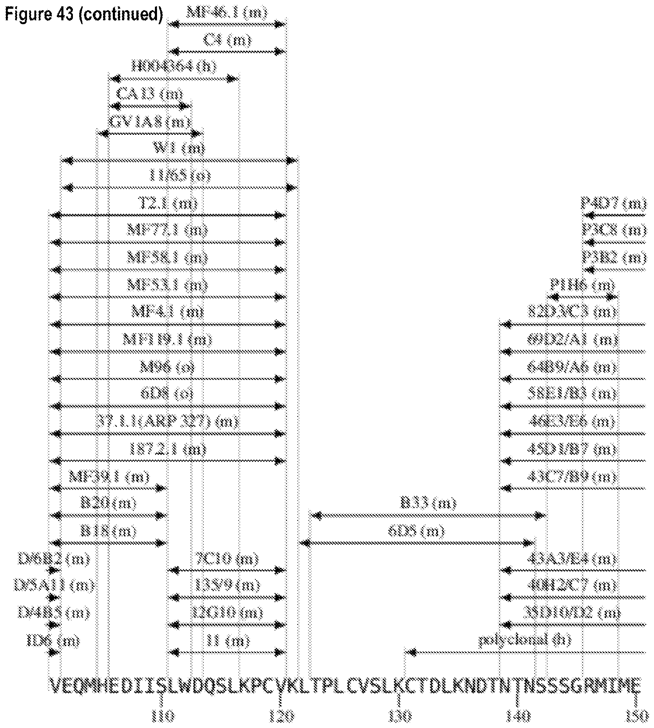

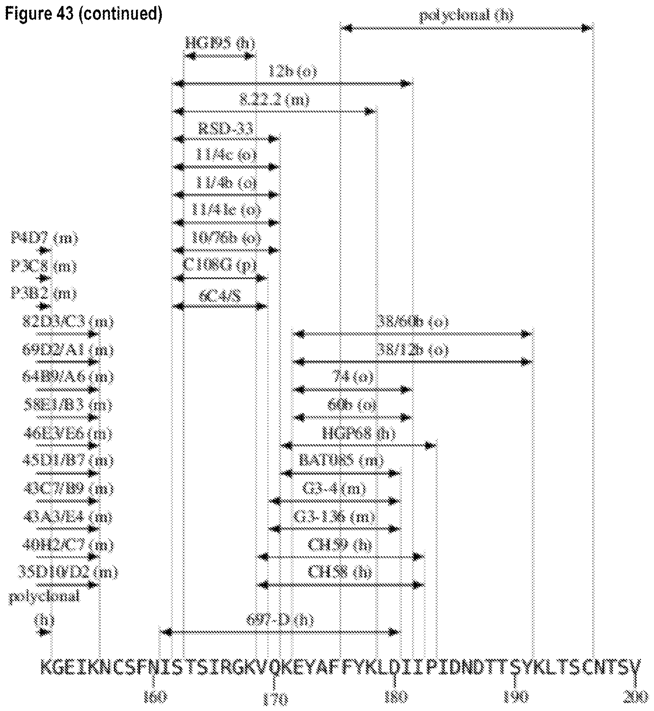

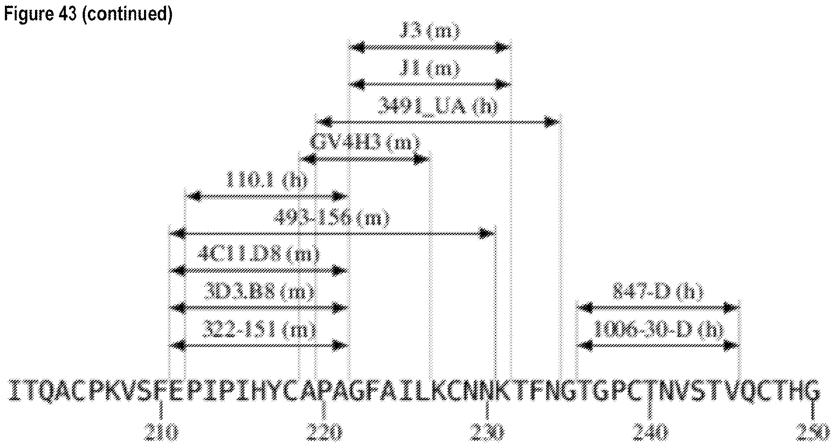

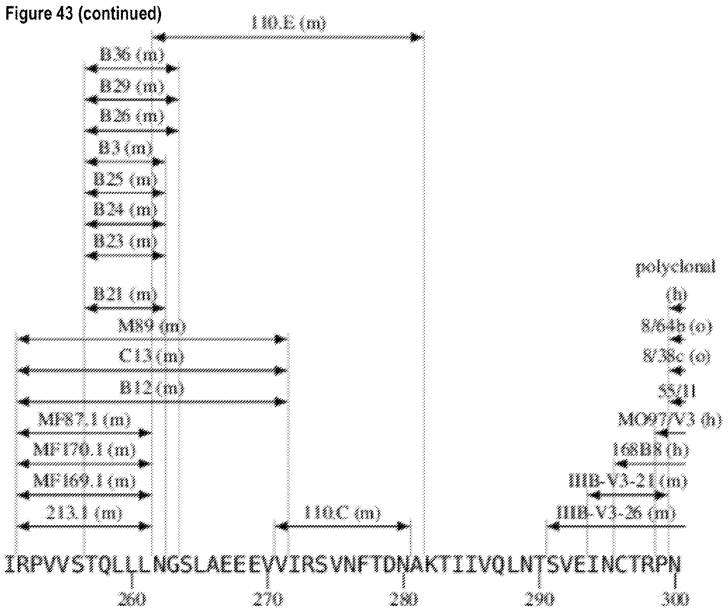

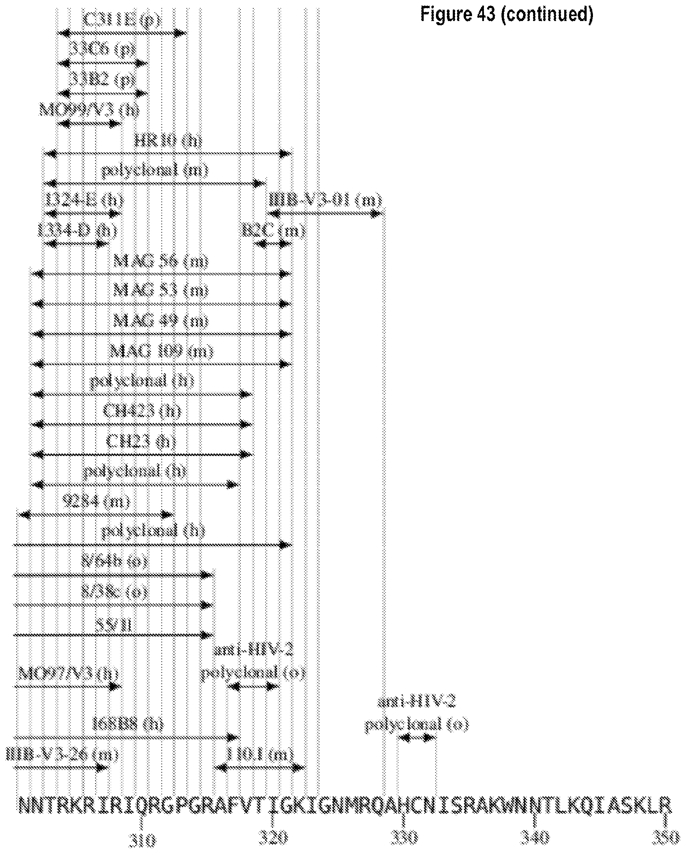

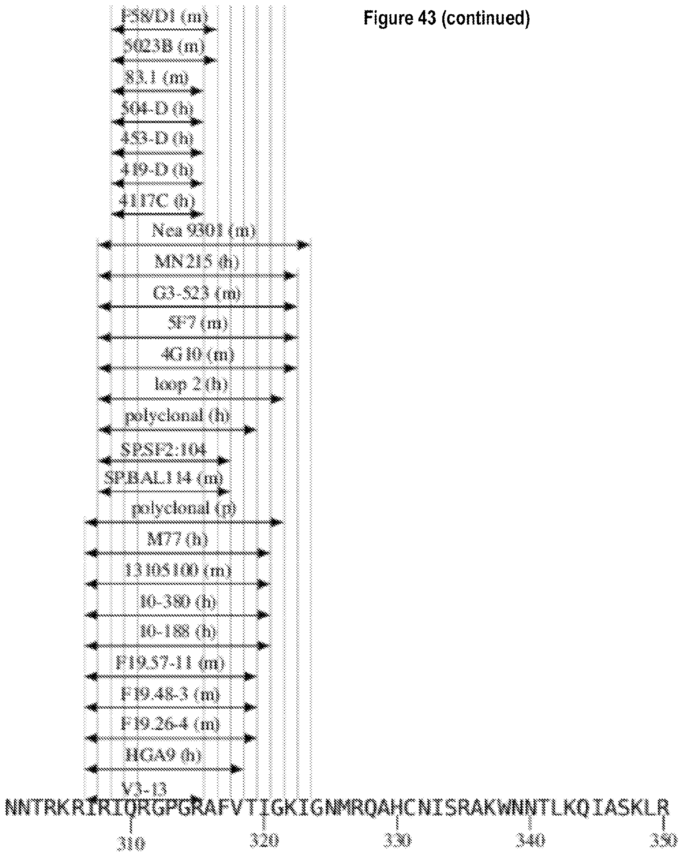

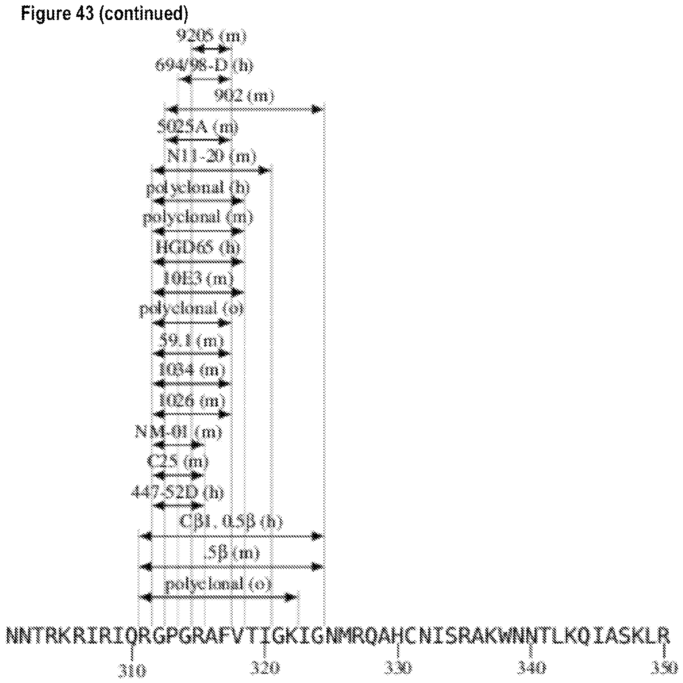

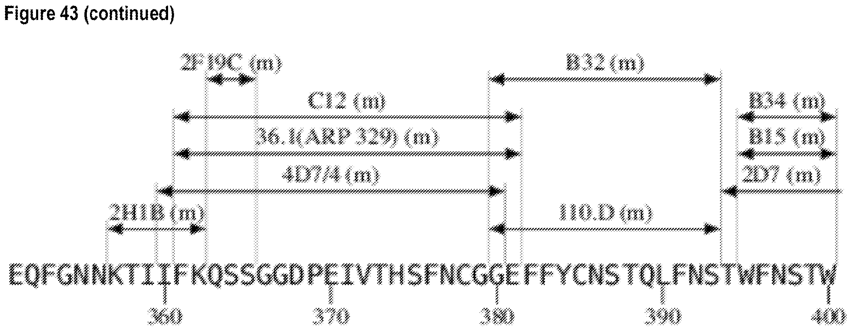

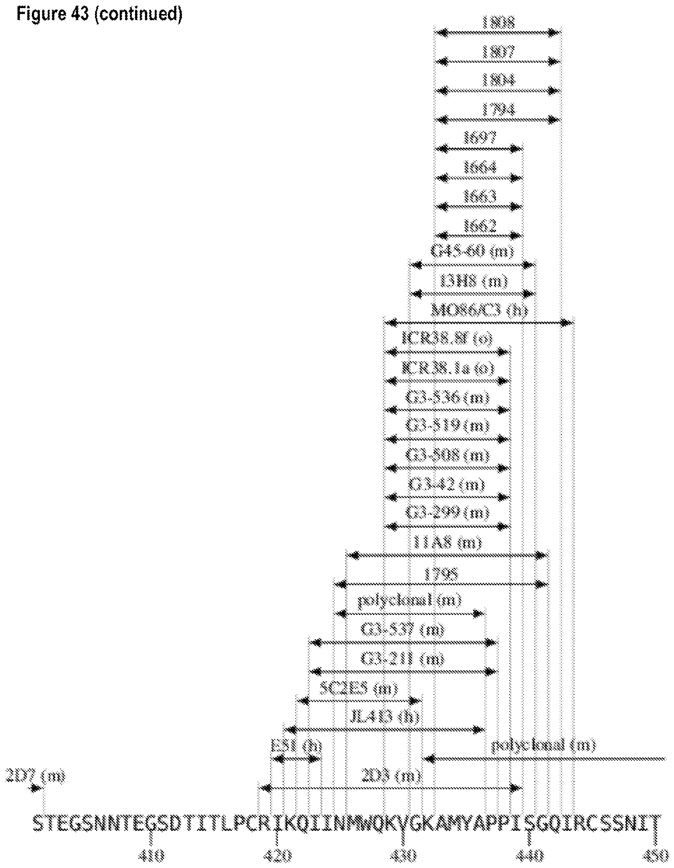

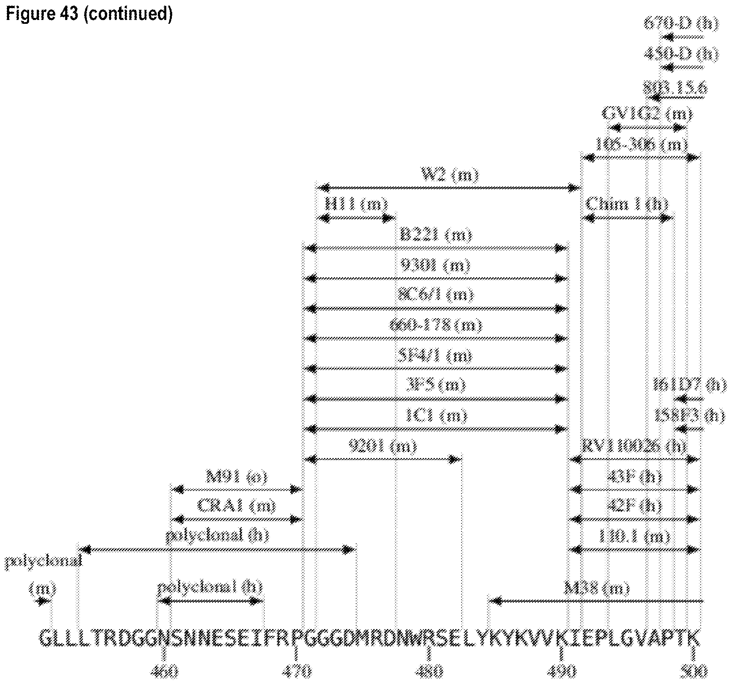

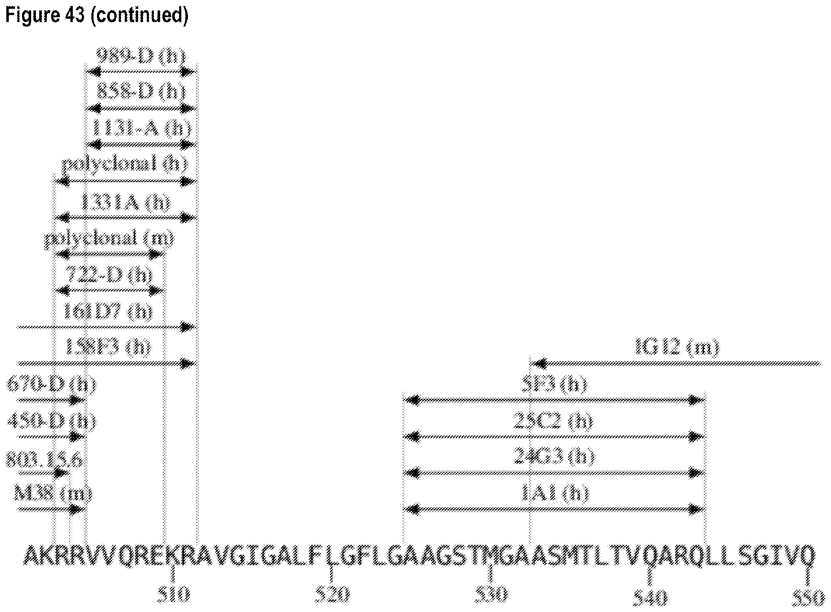

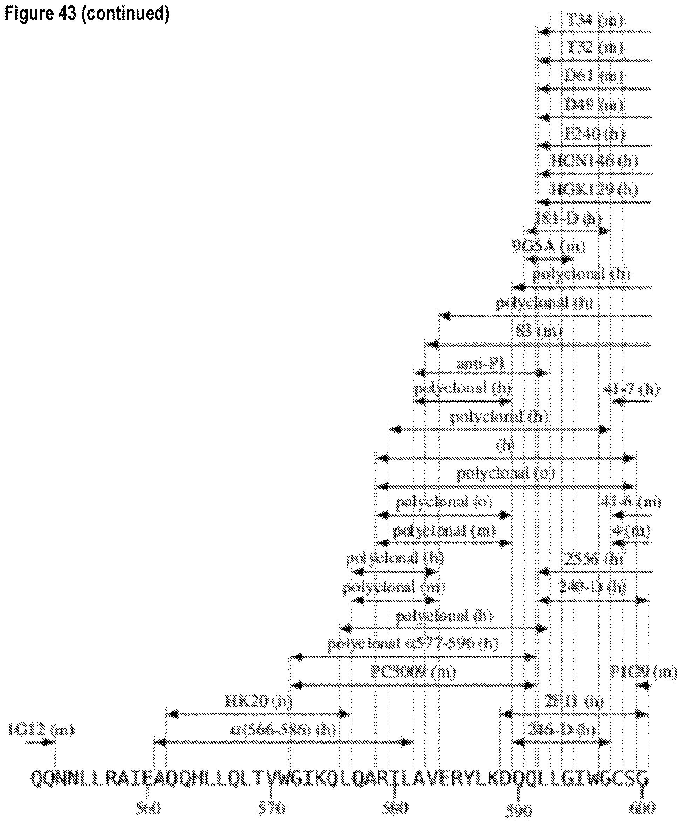

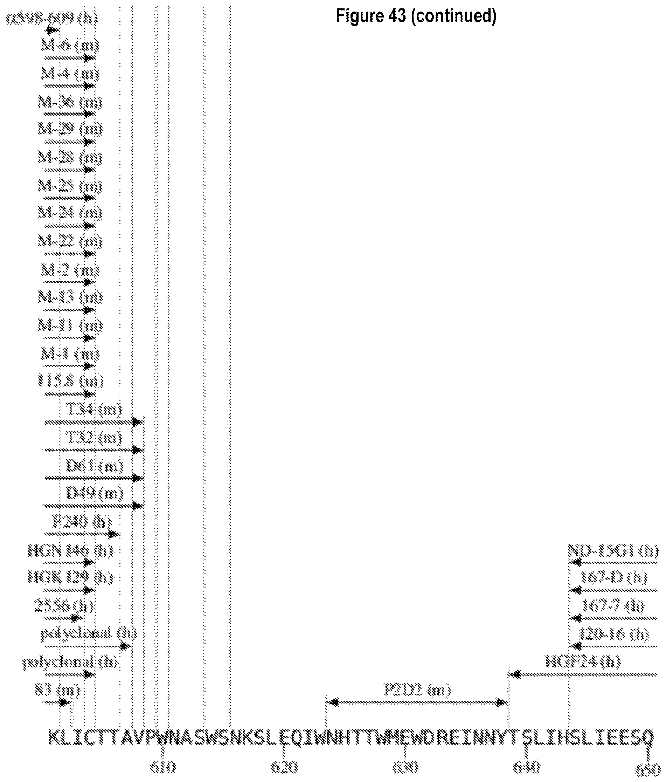

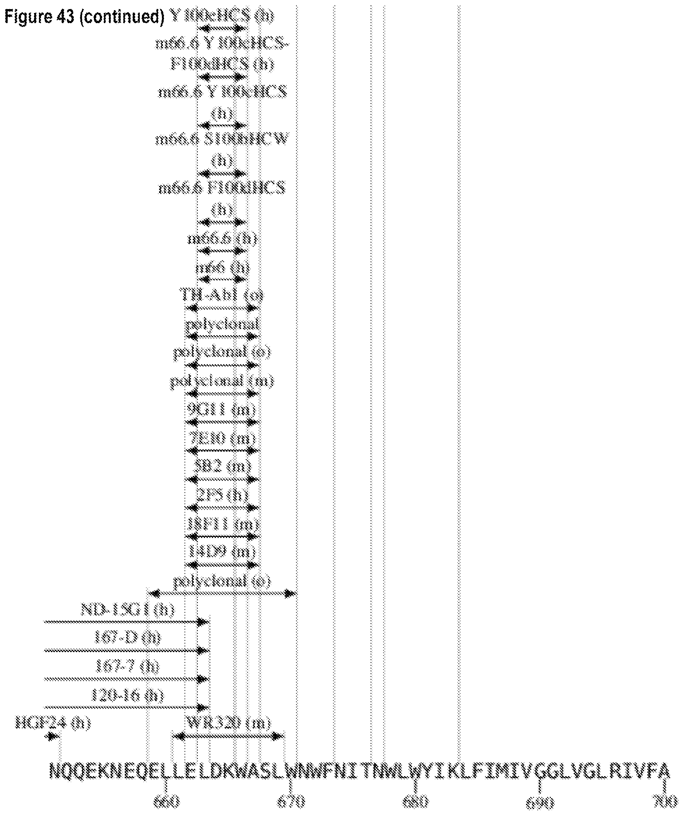

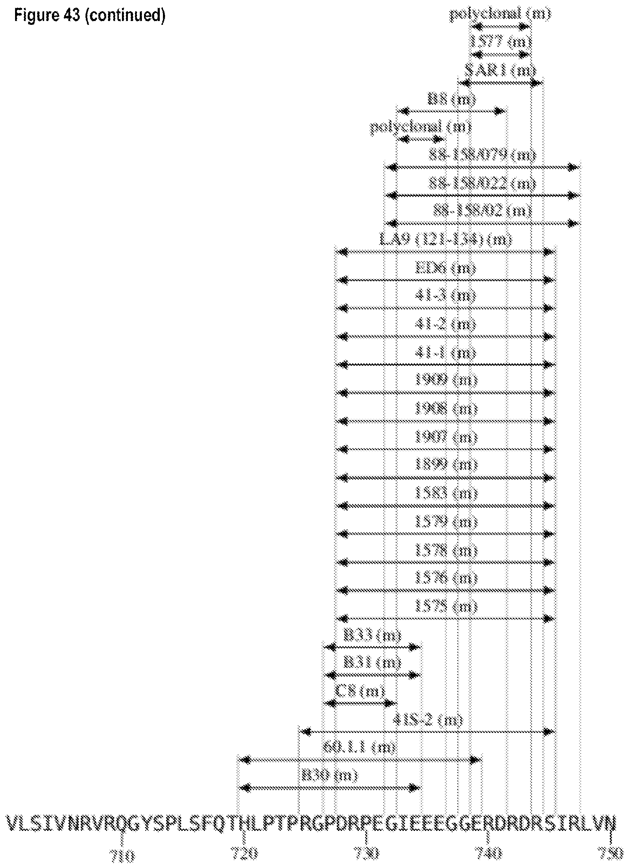

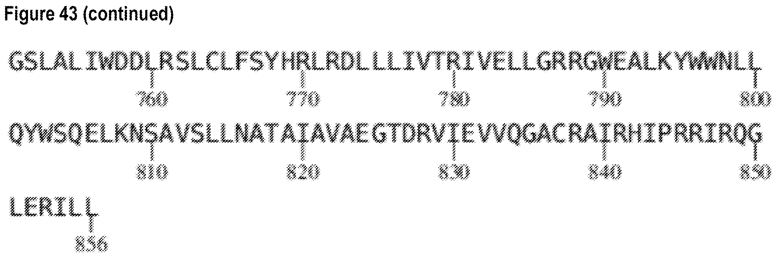

Figure 43 is an image of a portion of the October 19, 2017 version of the LANL gp160 AB Epitope Map, *available at* https://www.hiv.lanl.gov/content/immunology/maps/ab/ gp160.html.

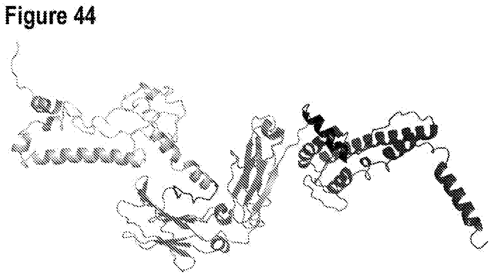

Figure 44 is an image of the predicted structure of a recombinant polypeptide having the amino acid sequence set forth in SEQ ID NO: 91, wherein the light grey portion comprises the HIV gp120 epitopes, the medium grey comprises the and antibody Fc domain, the dark grey comprises the HIV gp41 epitopes, and the black comprises the linkers.

### DETAILED DESCRIPTION

HIV-1 expresses the envelope proteins (gp120 and gp41) as a trimeric structure with gp41 embedded in the membrane and gp120 sitting on top of the respective gp41 proteins. Various embodiments of the invention include several distinct advantages for diagnostic use over prior art proteins. Some aspects of the embodiments relate to improvements created through the use of mammalian cells, which allow for correct post-translational modification that is crucial to epitopes, in particular to gp120 epitopes, but that is not reproducible in either yeast (*e.g.*, *Saccharomyces* or *Pichia)* or *E. coli.* Various aspects of the described embodiments relate to the discovery that recombinant HIV envelope polypeptides that are produced in mammalian cells and that lack a transmembrane domain display improved characteristics relative to full length recombinant HIV envelope proteins.

These polypeptides were designed to better emulate native HIV envelope structure, and to improve solubility and yield of recombinant HIV envelope polypeptides based on the fact that the yeast versions made to date and used by many diagnostic companies require harsh detergents to solubilized them (*e.g*., sodium dodecyl sulfate) and include large, irrelevant fusion proteins to aid expression (*e.g.,* superoxide dismutase). HIV polypeptides produced in mammalian cells adopt native conformation. The use of various fusion domains, as described herein, also improves HIV envelope solubility and stability and aids in purification.

The structure of the native HIV gp120/gp41 protein is dependent in part on the embedding of three transmembrane domains of three gp41 subunits into a lipid bilayer, and whether a recombinant HIV gp120 or gp41 polypeptide that lacks a transmembrane domain could fold into a native-like state was unknown and unpredictable. The majority of the polypeptides described herein are soluble in the absence of detergent and therefore capable of folding into native-like three-dimensional conformations.

The native HIV gp120/gp41 protein forms hetero-oligomeric quaternary structure. The quaternary structure of the native HIV gp120/gp41 protein is defined in part by its orientation in a membrane, which is fixed by the transmembrane helices of the gp41 subunit. Recombinant HIV gp120/gp41 polypeptides that lack a transmembrane helix lack a component to orient the monomers.

### I. POLYPEPTIDES

Various embodiments of the invention include a polypeptide comprising at least one epitope of the HIV gp120 protein, and at least one epitope of the HIV gp41 protein. The HIV envelope protein is preferably the HIV-1 envelope protein, which is more infectious and virulent than HIV-2, and it causes the majority of HIV infections worldwide. In some embodiments, the HIV envelope protein is the HIV-2 envelope protein.

The polypeptides featured in the embodiments described herein are all recombinant polypeptides, *i.e.,* they comprise two or more amino acid sequences that do not co-exist in naturally-occurring polypeptides. The terms "polypeptide" and "recombinant polypeptide" are therefore used interchangeably herein.

The sole proteins on the surface of an intact HIV virion are the envelope glycoproteins encoded by the *env* gene. The *env* gene product is an approximately 850 amino acid precursor protein called gp160, which is also referred to as the HIV envelope protein herein. Exemplary amino acid sequences of the HIV envelope protein are set forth in SEQ ID NO:39-62, and others may be found, for example, at https://www.hiv.lanl.gov/components/sequence/HIV/search/ search.html or at https://www.hiv.lanl.gov/content/sequence/NEWALIGN/align.html (*e.g.*, by selecting the "Alignment type" as "Web (all complete sequences)" and the "Year" as "2016"). The amino acid sequences of HIV envelope proteins may also be identified, for example, by searching the NCBI's Protein database for "gp160" or "HIV envelope protein" (*e.g.*, at https://www.ncbi.nlm.nih.gov/protein).

The term "polypeptide" refers to a molecule having an amino acid sequence of any length, although the term "polypeptide" as used in the claims and embodiments described herein requires a sufficiently long enough amino acid sequence to contain an HIV envelope protein epitope (*i.e.*, at least 8 amino acids) and a fusion protein. The term "domain" as used herein similarly refers to an amino acid sequence that is sufficiently long to fold independently. A domain may contain an epitope (*i.e.*, at least 8 amino acids), though a domain need not include an epitope. A transmembrane domain may include 8 or more amino acids, and yet a transmembrane domain may not contain an epitope, for example, if the domain is not antigenic.

A polypeptide according to the embodiments herein typically contains 200 to 1000 amino acids, such as about 200 to about 400, about 300 to about 500, about 400 to about 600, about 500 to about 700, about 600 to about 800, about 700 to about 900, or about 800 to about 1000 amino acids. The amino acids of a polypeptide are typically connected by peptide bonds, *e.g.,* because these polypeptides are typically produced by the translation of RNA on a ribosome.

A polypeptide of the embodiments typically has a molecular weight of about 20,000 to about 100,000 daltons, such as about 20,000 to about 40,000, about 30,000 to about 50,000, about 40,000 to about 60,000, about 50,000 to about 70,000, about 60,000 to about 80,000, about 70,000 to about 90,000, or about 80,000 to about 100,000 daltons.

A polypeptide of the embodiments typically has an isoelectric point (pl) of about 5 to about 10, such as about 5.5 to about 8.5, about 5.0 to about 7.0, about 6.0 to about 8.0, about 7.0 to about 9.0, about 5.0 to about 6.0, about 5.5 to about 6.5, about 6.0 to about 7.0, about 6.5 to about 7.5, about 7.0 to about 8.0, about 7.5 to about 8.5, about 8.0 to about 9.0, about 8.5 to about 9.5, or about 9.0 to about 10.0.

Polypeptides of the embodiments lack the gp41 transmembrane domain, *i.e.,* every polypeptide lacks the amino acid sequences encoded by any one of SEQ ID NO:63-72. A polypeptide of an embodiment may lack an amino acid sequence having at least 60%, 67%, 75%, 80%, 85%, 90%, or 95% sequence identity with an amino acid sequence encoded by SEQ ID NO:63-72. A polypeptide of an embodiment may lack an amino acid sequence that embeds in a biological membrane (*i.e.*, a lipid bilayer). The probability that an amino acid sequence may embed in a biological membrane may be determined using any number of different methods known in the art, which include, for example, calculations based on the E_{z} Depth-dependent Potential (Senes et al., J. Molecular Biology 366(2):436 (2007)).

A polypeptide of the invention may lack the gp120/gp41 cleavage site of a full-length HIV envelope protein.

### A. Epitopes of the HIV envelope protein

Embodiments include a polypeptide comprising at least four epitopes of the HIV envelope protein. A polypeptide as disclosed herein may include at least 4, 5, 6, 7, 8, 9, or 10 epitopes of the HIV envelope protein. A polypeptide may include a plurality of epitopes of the HIV envelope protein. A polypeptide may include a plurality of epitopes of the HIV envelope protein, wherein the plurality of epitopes comprises at least 4, 5, 6, 7, 8, 9, or 10 epitopes.

An "epitope", as the term is used in the specification and claims, refers to a sequence of at least 8 consecutive amino acids that can specifically bind the antigen-binding site of an antibody. An epitope may comprise, for example, at least 9, 10, 11, 12, 13, 14, 15, 16, 17, 18, 19, or 20 consecutive amino acids.

An epitope may consist of an amino acid sequence of at least 8, 9, 10, 11, 12, 13, 14, 15, 16, 17, 18, 19, or 20 consecutive amino acids found in any one of SEQ ID NO:39-62, which are sequences of HIV envelope proteins, so long as the sequence does not include the N- or C-terminus of a transmembrane domain, which are identified as SEQ ID NO:63-72. A polypeptide of an embodiment may comprise a plurality of epitopes, wherein each epitope of the plurality consists of an amino acid sequence of at least 8, 9, 10, 11, 12, 13, 14, 15, 16, 17, 18, 19, or 20 consecutive amino acids found in any one of SEQ ID NO:39-62, so long as the sequence does not include the N- or C-terminus of a transmembrane domain, which are identified as SEQ ID NO:63-72. Each epitope of a plurality of epitopes may have a different amino acid sequence or each epitope may have the same amino acid sequence. A polypeptide of an embodiment may comprise at least 1, 2, 3, 4, 5, 6, 7, 8, 9, or 10 epitopes of an HIV envelope protein, wherein each epitope consists of an amino acid sequence of at least 8, 9, 10, 11, 12, 13, 14, 15, 16, 17, 18, 19, or 20 consecutive amino acids found in any one of SEQ ID NO:39-62, so long as the sequence does not include the N- or C-terminus of a transmembrane domain, which are identified as SEQ ID NO:63-72.

Many variants of HIV envelope proteins are known. Accordingly, an epitope may consist of an amino acid sequence having at least about 80%, 85%, 90%, 95%, 96%, 97%, 98%, or 99% sequence identify with at least 8, 9, 10, 11, 12, 13, 14, 15, 16, 17, 18, 19, or 20 consecutive amino acids found in any one of SEQ ID NO:39-62, which are sequences of HIV envelope proteins, so long as the sequence does not include the N- or C-terminus of a transmembrane domain, which are identified as SEQ ID NO:63-72. A polypeptide of an embodiment may comprise a plurality of epitopes, wherein each epitope of the plurality consists of an amino acid sequence having at least about 80%, 85%, 90%, 95%, 96%, 97%, 98%, or 99% sequence identify with at least 8, 9, 10, 11, 12, 13, 14, 15, 16, 17, 18, 19, or 20 consecutive amino acids found in any one of SEQ ID NO:39-62, so long as the sequence does not include the N- or C-terminus of a transmembrane domain, which are identified as SEQ ID NO:63-72. Each epitope of a plurality of epitopes may have a different amino acid sequence or each epitope may have the same amino acid sequence. A polypeptide of an embodiment may comprise at least 1, 2, 3, 4, 5, 6, 7, 8, 9, or 10 epitopes of an HIV envelope protein, wherein each epitope consists of an amino acid sequence having at least about 80%, 85%, 90%, 95%, 96%, 97%, 98%, or 99% sequence identify with at least 8, 9, 10, 11, 12, 13, 14, 15, 16, 17, 18, 19, or 20 consecutive amino acids found in any one of SEQ ID NO:39-62, so long as the sequence does not include the N- or C-terminus of a transmembrane domain, which are identified as SEQ ID NO:63-72.

In embodiments wherein a polypeptide comprises more than four epitope of the HIV envelope protein, the epitopes of the HIV envelope protein may be the same epitope or a different epitope.

A polypeptide (or a plurality of epitopes) may include epitopes found in different HIV envelope proteins (*e.g.*, different amino acid sequences selected from SEQ ID NO:39-62), or the polypeptide (or plurality of epitopes) may include epitopes that are each found in the same HIV envelope protein.

Epitopes of the HIV envelope protein are well known. Epitopes of the HIV envelope protein may be selected, for example, from the LANL gp160 AB Epitope Map, *available at* https://www.hiv.lanl.gov/content/immunology/maps/ab/gp160.html. A portion of the October 19, 2017 version of the LANL gp160 AB Epitope Map is reproduced in Figure 43, and various embodiments of the invention feature epitopes described in the LANL gp160 AB Epitope Map.

Various aspects of the invention relate to polypeptides containing novel epitopes of the HIV envelope protein. In some embodiments, the polypeptide comprises at least one epitope of the HIV envelope protein, wherein the at least one epitope includes an epitope that is not identified in the LANL gp160 AB Epitope Map. A polypeptide may include, for example, epitopes or a plurality of epitopes wherein each epitope is a randomly-selected amino acid sequence of at least 8 consecutive amino acids found in any one of SEQ ID NO:39-62.

An epitope may be an epitope of the HIV gp120 protein or the HIV gp41 protein. A polypeptide may comprise an epitope of the HIV gp120 protein and/or the HIV gp41 protein. A plurality of epitopes may comprise epitopes of the HIV gp120 protein and/or the HIV gp41 protein.

An epitope of a polypeptide may be flanked by the amino acid sequences of a native HIV envelope protein. For example, a polypeptide may comprise at least two epitopes of an HIV envelope protein, wherein two epitopes of the at least two epitopes are connected by the native amino acid sequence of an HIV envelope protein.

A polypeptide may comprise at least two epitopes of an HIV envelope protein, wherein two epitopes of the at least two epitopes are connected by an amino acid sequence that is not found in naturally-occurring HIV envelope proteins. For example, a polypeptide may comprise at least 2, 3, 4, 5, 6, 7, 8, 9, or 10 epitopes, wherein 2, 3, 4, 5, 6, 7, 8, 9, or 10 of the at least 2, 3, 4, 5, 6, 7, 8, 9, or 10 epitopes are connected by amino acid sequences that are not found in naturally-occurring HIV envelope proteins. A polypeptide may comprise at least 2, 3, 4, 5, 6, 7, 8, 9, or 10 epitopes, wherein two epitopes of the at least 2, 3, 4, 5, 6, 7, 8, 9, or 10 epitopes are connected by an amino acid sequence found in a naturally-occurring HIV envelope protein.

In some embodiments, a polypeptide comprises a plurality of epitopes of the HIV envelope protein, wherein each epitope of the plurality of epitopes is connected to another epitope of the plurality of epitopes by an amino acid sequence that is not encoded by HIV. The terms "linker" or "spacer", may be used herein to refer to said amino acid sequences connecting at least two epitopes of the recombinant polypeptides described herein.

### 6. Linkers

Each epitope of a plurality of epitopes may be connected to another epitope of the plurality of epitopes by a linker or spacer. Each epitope of a plurality of epitopes may be connected to one or two other epitopes of the plurality of epitopes by a linker or spacer. A linker or spacer refers to a designed amino acid sequence, which is typically connected by peptide bonds and which either has a defined secondary structure or is intrinsically disordered. The terms "linker" and "spacer" are used interchangeably herein. A linker may comprise an amino acid sequence that forms an alpha helix or a beta strand (*e.g.*, wherein the beta strand is combined with other beta strands to form a beta sheet). A linker may comprise an alpha helix or a beta sheet.

A linker may consist of a sequence of consecutive amino acids that have a high propensity to form an alpha helix (*e.g*., alanine), which may optionally include an N-terminal helix capping motif (*e.g.*, serine-proline-glutamate) and/or alternately-charged amino acids at i and i+3/4 positions (*e.g.*, glutamate at i and lysine at either i+3 or i+4). An exemplary linker that has a high propensity to form an alpha helix is set forth in SEQ ID NO:73 (AEAAAKEAAAKA).

A linker may consist of a sequence of consecutive amino acids that have a high propensity to form a beta strand, such as "beta-branched" amino acids (*e.g.*, valine, isoleucine, threonine), although other sequences of consecutive amino acids that have a high propensity to form a beta strand are known in the art and may be selected, for example, to design a beta sheet. An exemplary linker that has a high propensity to form a beta strand is set forth in SEQ ID NO:74 (TWIQNPGTKWYQNPGTKIYT). In some embodiments, a polypeptide comprises a beta sheet, wherein at least two beta strands of the beta sheet are connected by an amino acid sequence comprising an epitope of the HIV envelope protein. For example, a polypeptide may comprise a beta sheet comprising at least three beta strands wherein a first beta strand is connected to a second beta strand by an amino acid sequence comprising a first epitope of the HIV envelope protein and a third beta strand is connected to a fourth beta strand by a second epitope of the HIV envelope protein (*i.e.*, wherein the first, second, third, and fourth beta strands make up either three or four beta strands (*e.g*., the third beta and either the first or second beta strand are the same beta strand, or each of the first, second, third, and fourth beta strands are different beta strands), and wherein the first epitope and second epitope may optionally have the same or different amino acid sequences).

An intrinsically disordered linker may consist of a sequence of consecutive amino acids that have a high degree of structural disorder. Intrinsically disordered linkers typically include at least one glycine, which lacks an R-group and thus has more accessible conformations than all other naturally-occurring amino acids, and/or at least one proline, which has an R-group that forms a ring with its backbone N and thus has fewer accessible conformations than all other naturally-occurring amino acids. Glycine increases structural disorder by introducing entropy into the secondary structure of the polypeptide, and proline increases structural disorder by introducing entropy into the secondary structure of the polypeptide. Intrinsically disordered linkers may also include other small amino acids (*e.g.*, serine, alanine) and hydrophilic amino acids. The amino acids of an intrinsically disordered linker may be selected, for example, from glycine, proline, alanine, serine, asparagine, aspartate, glutamine, glutamate, lysine, and arginine. Exemplary intrinsically disordered linkers include the amino acid sequences set forth in SEQ ID NO:75 (SGSGASGS), SEQ ID NO:76 (PSGP), SEQ ID NO:109 (GGGGSGGGGSGGGGS) or SEQ ID NO:110 (GGGGSGGGGSGGGGSGGGGSGGGGS), although the precise amino acid sequence of an intrinsically disordered linker is not particularly limiting.

A flexible linker may consist of a sequence of consecutive amino acids that typically include at least one glycine, at least one proline and at least one serine. Exemplary flexible linkers include the amino acid sequences set forth in SEQ ID NO:111 (GGGPS), although the precise amino acid sequence of an flexible linker is not particularly limiting.

Two epitopes of a plurality of epitopes (or of at least 2, 3, 4, 5, 6, 7, 8, 9, or 10 epitopes) may be connected by a linker, wherein the linker comprises an alpha helix, a beta strand, an intrinsically disordered or a flexible amino acid sequence. Each epitope of a plurality of epitopes (or of at least 2, 3, 4, 5, 6, 7, 8, 9, or 10 epitopes) may be connected to another epitope of the plurality of epitopes (or of the at least 2, 3, 4, 5, 6, 7, 8, 9, or 10 epitopes) by a linker, wherein the linker comprises an alpha helix, a beta strand, an intrinsically disordered or a flexible amino acid sequence.

A linker typically is not found in naturally-occurring HIV envelope proteins, and a linker is typically not found in nature, although some naturally-occurring amino acid sequences may serve as suitable linkers or may be used to design suitable linkers.

A linker may include about 1 to 30 amino acids, such as about 2 to 25, about 3 to 20, about 4 to 16, or about 1, 2, 3, 4, 5, 6, 7, 8, 9, 10, 11, 12, 13, 14, 15, 16, 17, 18, 19, or 20 amino acids.

### C. Fusion proteins

The polypeptides of the embodiments typically comprise a fusion protein, which comprises an amino acid sequence that is not found in naturally-occurring HIV envelope proteins. A fusion protein may comprise a region that is not encoded by HIV, said region selected from the group consisting of a Fc domain of an antibody. The fusion protein is preferably a sequence of at least 20 amino acids, such as at least 30, 40, 50, 60, 70, 80, 90, 100, 110, 120, 130, 140, 150, 160, 170, 180, 190, or 200 amino acids or about 20 to 500, about 50 to about 400, or about 75 to about 250 amino acids, although the precise length of the fusion protein is not particularly limiting.

A "fusion protein" as the term is used herein does not refer to a discrete protein that is separate from a polypeptide. The term "fusion protein" instead refers to an amino acid sequence comprising structural and/or functional characteristics that are typically useful for protein expression, purification, oligomerization, and/or stability. A gene encoding a polypeptide typically encodes a fusion protein in frame with the rest of the polypeptide without stop codons between the fusion protein and other portions of the polypeptide; a fusion protein is typically translated from mRNA as part of the polypeptide; and a fusion protein is typically connected to the other portions of a polypeptide by one or more native peptide bond(s).

A fusion protein typically has functional properties. For example, a fusion protein may function to oligomerize a polypeptide with another polypeptide. In some embodiments, a fusion protein simply increases the stability of the polypeptide, *e.g.,* a polypeptide may include an antibody Fc domain, which may increase the stability of the polypeptide. Stability may refer to the degradation of the polypeptide, the folding of the polypeptide, and/or the aggregation of the polypeptide, *e.g.,* and a fusion protein may increase the half-life of a polypeptide relative to degradation, increase the stability of the native fold of a polypeptide (or oligomeric polypeptide), and/or decrease the propensity of the polypeptide to form aggregates.

A fusion protein typically has a defined secondary structure and a defined tertiary structure and/or quaternary structure. The fusion protein may include a portion of an antibody Fc domain, for example, which has a defined secondary structure and tertiary structure, but that lacks quaternary structure (*e.g.*, because the Fc domain fragment lacks a hinge region and other elements that allow for an antibody to form dimers or higher-order oligomers).

A fusion protein may exist as a monomer, *i.e.,* the fusion protein may have a high dissociation constant for self-association when folded into its native conformation. A high dissociation constant (K_{D}) may be, for example, >10 mM, >1 mM, or > 100 µM as determined, for example, in phosphate-buffered saline at pH 7.

A fusion protein may comprise an oligomerization domain. An oligomeric polypeptide, such as a dimeric polypeptide, may be superior for developing antibodies against the native gp120/gp41 complex because the native gp120/gp41 exists as a trimer of hetero-dimers. Oligomerization domains are also useful to orient polypeptides thereby replicating native-like quaternary structure.

An exemplary oligomerization domain includes the amino acid sequence of an antibody Fc domain. An antibody Fc domain may increase the expression and/or secretion of a polypeptide in an expression cell. The use or omission of the hinge region may form dimeric or monomeric Fc fusions, respectively.

An exemplary fusion protein is the mouse IgG1 Fc domain or the mouse IgG2 Fc domain, which lacks the hinge region and therefore does not oligomerize.

A monomeric mouse IgG2 Fc domain may have the amino acid sequence set forth in SEQ ID NO:77 (APNLLGGPSVFIFPPKIKDVLMISLSPIVTCVVVDVSEDDPDVQISWFVNNVEVHTAQTQTHREDYNSTLR VVSALPIQHQDWMSGKEFKCKVNNKDLPAPIERTISKPKGSVRAPQVYVLPPPEEEMTKKQVTLTCMVT DFMPEDIYVEWTNNGKTELNYKNTEPVLDSDGSYFMYSKLRVEKKNWVERNSYSCSVVHEGLHNHHTT KSFSRTPGK) or an amino acid sequence having at least about 95%, 96%, 97%, 98%, or 99% sequence identity with the amino acid sequence set forth in SEQ ID NO:77.

A monomeric mouse IgG1 Fc domain may have the amino acid sequence set forth in SEQ ID NO:80 (VPEVSSVFIFPPKPKDVLTITLTPKVTCVVVDISKDDPEVQFSWFVDDVEVHTAQTQPREEQFNSTFRSV SELPIMHQDWLNGKEFKCRVNSAAFPAPIEKTISKTKGRPKAPQVYTIPPPKEQMAKDKVSLTCMITDFFP EDITVEWQWNGQPAENYKNTQPIMDTDGSYFVYSKLNVQKSNWEAGNTFTCSVLHEGLHNHHTEKSLS HSPG) or an amino acid sequence having at least about 95%, 96%, 97%, 98%, or 99% sequence identity with the amino acid sequence set forth in SEQ ID NO:80.

Exemplary fusion proteins may comprise Fc domains that lack an oligomerization domain having the amino acid sequence set forth in SEQ ID NO:77 or SEQ ID NO:80, and therefore the recombinant polypeptide is a monomer.

Fc domains may also aid the purification of a polypeptide as methods of purifying polypeptides comprising Fc domains are well known.

Fusion proteins of the embodiments do not include superoxide dismutase, which commonly used in the preparation of recombinant HIV envelope proteins, *i.e.,* polypeptides of the invention lack an amino acid sequence corresponding to superoxide dismutase.

### D. Leader Peptide Sequences

Polypeptides disclosed herein typically comprise a leader peptide sequence to favor translocation of the polypeptide across the cell membrane of an expression vector, such as a mammalian cell, especially a human cell. A polypeptide may nevertheless lack a leader peptide sequence, for example, if the leader peptide sequence is cleaved from the polypeptide by enzymatic or chemical cleavage. Synthetically-produced polypeptides may similarly lack a leader peptide sequence.

A leader peptide sequence is typically included at the N-terminus of a polypeptide. A leader peptide sequence is preferably sufficient to translocate the polypeptide outside of the cell surface membrane of a eukaryotic cell (*e.g.*, a mammalian cell, such as a human cell) following the translation of the polypeptide in the eukaryotic cell, although other sequence motifs of a polypeptide may also aid translocation.

An exemplary leader peptide sequence has the amino acid sequence set forth in SEQ ID NO:84 (MYRMQLLSCIALSLALVTNS), which is the human interleukin-2 leader peptide sequence. This well-characterized sequence is capable of translocating polypeptides out of both human cells and other mammalian cells.

In some embodiments, the leader peptide of the polypeptide disclosed herein is the fusion protein of the polypeptide. In some embodiments, the polypeptide comprises an epitope of HIV gp120 and an epitope of HIV gp41 and the polypeptide lacks the gp120/gp41 cleavage site.

A leader peptide sequence is typically not derived from the HIV envelope protein, although in some embodiments, the leader peptide sequence is the signal sequence of the HIV envelope protein.

### E. Affinity Tags

A polypeptide may optionally include an affinity tag. Affinity tags are useful for purification, and they may also be useful in assays that utilize a polypeptide. Exemplary affinity tags include polyhistidine, chitin binding protein, maltose binding protein, Strep-tag, glutathione-S-transferase, FLAG-tag, V5-tag, Myc-tag, HA-tag, NE-tag, AviTag, Calmodulin-tag, polyglutamate, S-tag, SBP-tag, Softag 1, Softag 3, TC tag, VSV-tag, Xpress tag, Isopeptag, SpyTag, SnoopTag, biotin carboxyl carrier protein, green fluorescent protein-tag, HaloTag, Nus-tag, and thioredoxin-tag, although the choice of affinity tag is not particularly limiting. A polypeptide may nevertheless lack an affinity tag, for example, if the affinity tag is removed after use or if the polypeptide is purified using a strategy that does not require an affinity tag. An exemplary affinity tag is polyhistidine, which typically includes an amino acid sequence comprising six consecutive histidines (SEQ ID NO:85). Another exemplary affinity tag is polyhistidine comprising between 4 and 8 consecutive histidines.

### F. Exemplary Polypeptide Sequences

The recombinant polypeptides featured in the embodiments described herein may comprise a leader peptide, a fusion protein, at least one epitope of the HIV gp120 protein and at least one epitope of the HIV gp41 protein wherein the recombinant polypeptide lacks a transmembrane domain and wherein the fusion protein comprises a region that is not encoded by HIV.

A polypeptide of the sort disclosed herein may have the amino acid sequence set forth in SEQ ID NO:14, SEQ ID NO:16, SEQ ID NO:21, SEQ ID NO:22, SEQ ID NO:23, SEQ ID NO:24, SEQ ID NO:25, SEQ ID NO:26, SEQ ID NO:27, SEQ ID NO:28, SEQ ID NO:29, SEQ ID NO:30, SEQ ID NO:31, SEQ ID NO:91, SEQ ID NO:92, SEQ ID NO:93, SEQ ID NO:94, SEQ ID NO:95, SEQ ID NO:96, SEQ ID NO:97, SEQ ID NO:98, SEQ ID NO:99, SEQ ID NO:100,SEQ ID NO:101, SEQ ID NO:102, SEQ ID NO:103, SEQ ID NO:104, SEQ ID NO:105, or SEQ ID NO:106. A polypeptide may have an amino acid sequence that has at least about 85%, 86%, 87%, 88%, 89%, 90%, 91%, 92%, 93%, 94%, 95%, 96%, 97%, 98%, 99%, or 99.5% sequence identity with the amino acid sequence set forth in SEQ ID NO:14, SEQ ID NO:16, SEQ ID NO:21, SEQ ID NO:22, SEQ ID NO:23, SEQ ID NO:24, SEQ ID NO:25, SEQ ID NO:26, SEQ ID NO:27, SEQ ID NO:28, SEQ ID NO:29, SEQ ID NO:30, SEQ ID NO:31 SEQ ID NO:91, SEQ ID NO:92, SEQ ID NO:93, SEQ ID NO:94, SEQ ID NO:95, SEQ ID NO:96, SEQ ID NO:97, SEQ ID NO:98, SEQ ID NO:99, SEQ ID NO:100,SEQ ID NO:101, SEQ ID NO:102, SEQ ID NO:103, SEQ ID NO:104, SEQ ID NO:105, or SEQ ID NO:106, i.e., wherein the complete amino acid sequence of the polypeptide is not a naturally-occurring amino acid sequence.

### II. IMMUNOBIOLOGY OF INFECTION WITH HIV-1 AND HIV-2

Infection with HIV leads the immune system to respond in one of a number of ways depending upon the health and genetic makeup of the host. In general, once HIV enters the host via infection of CD4+ T cells, it begins to replicate and produce new viral particles, which are released from lysed cells into the blood stream. On the surface of this enveloped viruses are the "Envelope Associated Proteins" gp120 and gp41 in sparsely interspersed trimeric spikes. The gp120 protein that sits on top of the gp41 protein, however, tends to be shed from the virus (likely strain dependent), and this shedding is believed to function as a decoy protein by some in the field. Thus the gp41 is exposed as the first initial surface target for the host antibody response. Indeed, the vast majority of HIV+ patients develop a strong antibody response to the gp41 moeity, and this is why past envelope proteins used by many diagnostic companies such as Env13, Env31 and Env70, which are expressed in yeast, consist essentially of the gp41 sequence with only a small fragment of gp120. For most patients infected with HIV and having mounted an antibody response, this protein will display antibody reactivity around 90-98% of the time. The small percentage of nonreactive patients displays a measurable antibody response to the gp120 and/or the p24 antigen.

### III. NUCLEIC ACIDS, CLONING CELLS, AND EXPRESSION CELLS

Embodiments described herein also include a nucleic acid comprising a nucleotide sequence encoding a polypeptide described herein. The nucleic acid may be DNA or RNA. DNA comprising a nucleotide sequence encoding a polypeptide described herein typically comprises a promoter that is operably-linked to the nucleotide sequence. The promoter is preferably capable of driving constitutive or inducible expression of the nucleotide sequence in an expression cell of interest. The precise nucleotide sequence of the nucleic acid is not particularly limiting so long as the nucleotide sequence encodes a polypeptide described herein. Codons may be selected, for example, to match the codon bias of an expression cell of interest (*e.g.*, a mammalian cell such as a human cell) and/or for convenience during cloning. DNA may be a plasmid, for example, which may comprise an origin of replication (*e.g.*, for replication of the plasmid in a prokaryotic cell).

Various aspects of the instant disclosure also relate to a cell comprising a nucleic acid comprising a nucleotide sequence that encodes a polypeptide described herein. The cell may be an expression cell or cloning cell. Nucleic acids are typically cloned in *E. coli,* although other cloning cells may be used. If the cell is an expression cell, the nucleic acid is optionally a nucleic acid of a chromosome, *i.e.,* wherein the nucleotide sequence is integrated into the chromosome, although then nucleic acid may be present in an expression cell, for example, as extrachromosomal DNA.

Various aspects of the instant disclosure include a cell comprising a nucleic acid comprising a sequence that encodes a polypeptide as described herein. The cell is typically an expression cell. The nature of the expression cell is not particularly limiting. Mammalian expression cells may allow for favorable folding, post-translational modifications, and/or secretion of a polypeptide, although other eukaryotic cells or prokaryotic cells may be used as expression cells. Exemplary expression cells include CHO, BHK, NS0, Sp2/0, COS, C127, HEK, HT-1080, PER.C6, HeLa, and Jurkat cells.

An expression cell of the invention is typically not yeast, which is commonly used as an expression cell for expressing recombinant HIV envelope proteins. The expression of HIV envelope proteins in yeast may result in misfolding, aggregation, and/or the accrual of aberrant post-translational modifications, which is mitigated by expressing the polypeptides described herein in mammalian expression cells (especially human expression cells).

### IV. ASSAYS AND REAGENTS

Embodiments include an immunoassay reagent comprising a polypeptide as described herein for assaying a composition containing an anti-HIV antibody. The immunoassay reagent may be bound to a solid support. A solid support may be, for example, a bead, membrane, microtiter plate, polypeptide chip, or the solid-phase of a chromatography column.

Various embodiments also include a device for assaying a composition containing an anti-HIV antibody. The device may be a device for determining whether a sample contains an anti-HIV antibody (*e.g.*, for determining whether the sample may be used in a transfusion or transplant into a human patient). Assays are well known and typically feature a solid support that aids in the separation of components that directly or indirectly bind the solid support from components that do not directly or indirectly bind the solid support. A solid support may be, for example, a bead, membrane, microtiter plate, polypeptide chip, or the solid-phase of a chromatography column. A device may comprise a polypeptide as described herein. The polypeptide is typically covalently or non-covalently bound to the solid support.

The term "direct" binding, as used herein, refers to the direct conjugation of a molecule to a solid support, *e.g.,* a gold-thiol interaction that binds a cysteine thiol of a polypeptide to a gold surface. The term "indirect" binding, as used herein, includes the specific binding of a polypeptide to another molecule that is directly bound to a surface, *e.g.,* a polypeptide may bind an antibody that is directly bound to a solid support thereby indirectly binding the polypeptide to the solid support. The term "indirect" binding is independent of the number of molecules between the polypeptide and the solid support so long as each interaction between the daisy chain of molecules is a specific or covalent interaction and a terminal molecule of the daisy chain is directly bound to the solid support.

The term "non-covalently bound," as used herein, refers to specific binding such as between an antibody and its antigen, a ligand and its receptor, or an enzyme and its substrate, exemplified, for example, by the biotin-streptavidin interaction. Specific binding generally refers to interactions with a dissociation constant (K_{D}) of less than about 100 µM, such as less than about 10 µM, about 1 µM, about 100 nM, or about 10 nM. Assays may also be devised in which the polypeptide never binds the solid support, such as in a competition assay.

In an exemplary assay, a polypeptide bound to a solid support is contacted with a sample suspected of containing an anti-HIV antibody, such as a blood, blood serum, blood plasma, saliva, or cheek swab sample. The solid support is then washed at a stringency that does not significantly disrupt binding between the polypeptide and any anti-HIV antibody (*e.g.*, a phosphate-buffered saline or similar wash). The solid support is then contacted with a "secondary" antibody that recognizes the HIV antibody (*e.g*., an anti-human IgG antibody), wherein the secondary antibody includes a detection label. The detection label may be a dye (*e.g.*, for visual detection), a fluorophore (*e.g.*, for fluorescence detection), an enzyme (*e.g.*, horseradish peroxidase; for visual or fluorescence detection of an amplified signal), a radiolabel, or any other detection label commonly-used in molecular biology or medical diagnostics. Different variations of this assay are readily apparent to those skilled in the art. For example, the antibodies of a sample may be immobilized on a solid support, the polypeptide may be contacted with the antibodies, and then the polypeptide may be detected using an antibody or ligand that recognizes the fusion protein or affinity tag of the polypeptide.

Embodiments also include a method of assaying a sample, comprising contacting a polypeptide as described herein with the sample. Additional embodiments include a method of determining whether a sample comprises an anti-HIV antibody, comprising contacting a polypeptide as described herein with the sample. Embodiments also include a method of assaying a sample, comprising contacting a polypeptide as described herein with the sample and determining the binding affinity between the antibody and either the. The sample may be a human sample, such as a bodily fluid obtained from a human. The sample may be, for example, blood, blood plasma, blood serum, saliva, or a cheek swab. The sample may comprise blood, blood plasma, blood serum, saliva, or cheek cells. The sample may be a sperm sample.

### VI. PHARMACEUTICAL COMPOSITIONS

Various embodiments of the invention relate to compositions comprising a polypeptide described herein. A composition may comprise a pharmaceutically-acceptable carrier and/or a pharmaceutically-acceptable excipient. The composition may be, for example, a vaccine.

Various embodiments of the invention relate to a composition comprising a polypeptide as described herein for use in a method of treating or preventing an HIV infection in a human patient comprising administering to the patient a composition comprising a polypeptide as described herein. The term "preventing" as used herein refers to prophylaxis, which includes the administration of a composition to a patient to reduce the likelihood that the patient will become infected with HIV relative to an otherwise similar patient who does not receive the composition. The term preventing also includes the administration of a composition to a group of patients to reduce the number of patients in the group who become infected with HIV relative to an otherwise similar group of patients who do not receive the composition.

Various embodiments of the invention relate to a vaccine for use in a method of treating or preventing an HIV infection in a human patient comprising administering to the patient a vaccine according to the embodiments described herein.

A patient may be infected with HIV, a patient may have been exposed to HIV, or a patient may present with an elevated risk for exposure to and/or infection with HIV.

### VII. METHODS OF SELECTING ANTIBODIES

Additional embodiments include a method for assaying a sample containing an antibody, comprising contacting the sample with a polypeptide described herein and determining the binding affinity between the antibody and the polypeptide. The binding affinity may be a relative binding affinity, *e.g.,* the binding affinity may be relative to the binding affinity of one or more different antibodies present in the sample or otherwise assayed using a substantively identical method. Similarly, the binding affinity may be a quantitative binding affinity, *e.g.*, the method may include determining a dissociation constant (K_{D}) within a desirable range of certainty, such as ± an order of magnitude, t50%, ±10%, etc. The method may be a method of antibody affinity maturation.

Embodiments also include a method of selecting an anti-HIV antibody from a plurality of antibodies. The method may comprise contacting a polypeptide as described herein with a composition containing the plurality of antibodies and identifying an antibody that has a high binding affinity to the polypeptide relative to other antibodies in the composition, thereby selecting the anti-HIV antibody. The method may be a method of selecting an anti-HIV antibody that specifically binds an antigenic determinant of a gp120 amino acid sequence or a gp41 amino acid sequence as described herein.

### VIII. METHODS OF SELECTING BIOLOGICAL SAMPLES

Disclosed is also a method of selecting biological samples from a supply of human biological samples comprising selecting from the supply those samples that comprise antibodies that form an antigen-antibody complex with a polypeptide as described herein. This is useful to identify an HIV positive sample for removal from the supply, particularly relevant when the supply is a blood supply. Those samples which are not selected can be employed for the preparation of blood-related products. By identifying an HIV positive sample, the method can also be useful in the enrichment of positive samples.

Disclosed is also a method of selecting biological samples from a supply of human biological samples comprising selecting from the supply those samples that do not comprise antibodies that form an antigen-antibody complex with the immunoassay reagent according to the subject invention. This is useful to identify biological samples useful for the preparation of blood-related products.

### EXEMPLIFICATION

### Example 1. Design of polypeptides comprising epitopes of the HIV envelope protein

Various polypeptides containing epitopes of the HIV envelope protein were designed. Polypeptides env_1 to env_4 were designed using the gp120 human B cell epitopes derived from the LANL HIV-1 gp160 Envelope Epitope Map (https://www.hiv.lanl.gov/content/immunology/maps/ab/gp160.html) stitched together using either alpha helical or beta sheet linker sequences. These sequences were also fused to a murine IgG CH2/CH3 region. In some versions, the IgG hinge region was included thereby allowing the formation of a dimer. Polypeptide env_5 was designed using the same SYN-gp120 sequence fused to the tetramerization motif of p53. Polypeptide env_6 was designed using the SYN-gp120 sequence fused to the trimerization motif of GCN4. Polypeptide env_7 was designed using the SYN-gp120 sequence fused to the trimerization motif of clathrin. Polypeptides env_8 and env_9 were designed using the human B cell epitopes from the LANL HIV gp160 epitope map corresponding to gp41 stitched together with alpha helical linkers designated by "SYN-gp41" and fused to the IgG CH2/CH3 domain with or without the hinge region. Polypeptides env_10 to env_17 were designed using an HIV-1 gp120 sequence derived from amino acid sequences from RCSB Protein Data Bank (PDB) entries 5TE4 and 5T33 fused to the IgG CH2/CH3 domain with or without a hinge region or the V3 loop of gp120. Polypeptides env_14 to env_17 further comprise a portion of the HIV-1 gp41 sequence derived from the amino acid sequences from RCSB Protein Data Bank (PDB) entry 5V8L fused to the C-terminus of the IgG CH2/CH3 domain following an alpha-helical linker. Polypeptides env_18 and env_19 were designed using the gp41 sequence derived from PDB entry 5V8L fused to the IgG CH2/CH3 domain with or without a hinge region, respectively. Polypeptide env_20 was a modification of polypeptide env_8 with one epitope region extended. Polypeptide env_21 was the same as polypeptide env_20 with the SYN-gp120 domain fused at the C-terminus. Polypeptide env_22 was a truncated gp41 sequence derived from NCBI Reference Sequence NP_579895 and fused to the SYN-gp120 sequence with an IgG CH2/CH3 domain in between the gp41 and gp120 sequences. Polypeptides env_23 and env_24 were derived from an HIV-1 envelope protein epitope and fused to the IgG CH2/CH3 domain at its C- or N-terminus, respectively. Polypeptides env_25 to env_27 were the Clade A, Clade C, and a consensus sequence corresponding to an HIV envelope protein epitope. Polypeptides env_28 and env_29 correspond to an HIV-2 envelope protein epitope fused to an IgG CH2/CH3 domain at its N- or C-terminus, respectively. Polypeptides env_30 and env_31 correspond to an HIV-1 envelope protein epitope fused to an IgG CH2/CH3 domain at its N- or C-terminus, respectively. Polypeptides env_32 and env_33 correspond to the same gp41 sequences used in polypeptide env_22 with an IgG CH2/CH3 domain fused at its N- or C-terminus, respectively. Polypeptide env_34 corresponds to the gp160 sequence derived from SEQ ID NO:10 of US Patent No. 6,284,248, which was used as a control for polypeptides env_35 to env_38. This sequence does not include the envelope transmembrane sequence and has the gp120/gp41 cleavage site mutated. Polypeptides env_35 to env 38 correspond to envelope sequences from clades A, C, AE, and a consensus sequence aligned with the sequences of polypeptide env_34. Polypeptide env_39 was derived from a HIV-1 gp120 Clade B envelope protein epitope fused to a truncated gp41 sequence derived from the NCBI Reference Sequence NP_579895 with an IgG CH2/CH3 domain in between the gp120 and gp41 sequences. Polypeptide env_40 corresponds to the same sequences of HIV-1 gp120 envelope, a truncated gp41 fused together with an IgG CH2/CH3 domain but with a longer linker. Polypeptide env_41 corresponds to a truncated gp41 sequence derived from the NCBI Reference Sequence NP_579895 fused at its C-terminus to a HIV-1 gp120 envelope protein epitope with an IgG CH2/CH3 domain in between the gp41 and gp120 sequences. Polypeptides env_42 to env_44 are similar to polypeptide env_40, but use a gp120 sequence from a Clade A, AE and AG strains, respectively. Polypeptide env_45 is similar to polypeptide env_40, but uses a consensus gp120 sequence. Polypeptide env_46 was derived from a HIV-1 gp120 Clade B envelope protein epitope fused to a HIV-1 capsid protein p24 sequence with an IgG CH2/CH3 domain in between the gp120 and p24 sequences. Polypeptide env_47 was derived from a a HIV-1 capsid protein p24 sequence fused to a HIV-1 gp120 Clade B envelope protein epitope with an IgG CH2/CH3 domain in between the p24 and gp120 sequences. Polypeptide env_48 is similar to polypeptide env_46 with selected epitopes of the p24 protein removed. Polypeptide env_49 is similar to polypeptide env_47 with selected epitopes of the p24 protein removed. Polypeptide env_50 is similar to polypeptide env_46 with selected p24 mutations. Polypeptide env_51 is similar to polypeptide env_47 with selected p24 mutations. Polypeptide env_52 is similar to polypeptide env_46 with select alanine substitutions in the p24 sequence. Polypeptide env_53 is similar to polypeptide env_47 with select alanine substitutions in the p24 sequence. Polypeptide env_54 corresponds to a HIV-1 gp41 sequence fused at its C-terminus to a HIV-1 gp120 protein with and IgG CH2/CH3 domain.

Each numbered "polypeptide" from env_1 to env_38 corresponds to a SEQ ID NO:1 to SEQ ID NO:38. Each numbered "polypeptide" from env_39 to env_54 corresponds to a SEQ ID NO:91 to SEQ ID NO:106.

### Example 2. Models of env recombinant polypeptides

The structures of polypeptides env_1 to env_38 and env_39 to env_54 described in Example 1 and having amino acid sequences set forth in SEQ ID NO:1-38 and SEQ ID NO:91-106, respectively, were predicted using Rosetta software within the Cyrus Biotech protein modeling tool or by manually stitching together known structures from the PDB using PyMol. Figures 2-40 and 44 were rendered in PyMol. Fusion proteins are shown in dark gray, and amino acid sequences of the HIV envelope protein (which comprise one or more epitopes) are shown in light gray.

Figure 2 is a model of a monomeric polypeptide that is representative of the monomeric subunits encoded by the amino acid sequences set forth in SEQ ID NO:1 and SEQ ID NO:3.

Figure 3 is a model of a monomeric polypeptide that is representative of the monomeric subunits encoded by the amino acid sequences set forth in SEQ ID NO:2 and SEQ ID NO:4.

Figure 4 is a model of a monomeric polypeptide that is representative of the monomeric subunit encoded by the amino acid sequence set forth in SEQ ID NO:5.

Figure 5 is a model of a monomeric polypeptide that is representative of the monomeric subunit encoded by the amino acid sequence set forth in SEQ ID NO:6.

Figure 6 is a model of a monomeric polypeptide that is representative of the monomeric subunit encoded by the amino acid sequence set forth in SEQ ID NO:7.

Figure 7 is a model of a monomeric polypeptide that is representative of the monomeric subunits encoded by the amino acid sequences set forth in SEQ ID NO:8 and SEQ ID NO:20.

Figure 8 is a model of a monomeric polypeptide that is representative of the monomeric subunit encoded by the amino acid sequence set forth in SEQ ID NO:9.

Figure 9 is a model of a monomeric polypeptide that is representative of the monomeric subunits encoded by the amino acid sequences set forth in SEQ ID NO:10, SEQ ID NO:11, and SEQ ID NO:12.

Figure 10 is a model of a dimeric polypeptide that is representative of the dimeric polypeptides formed from two monomeric polypeptides encoded by the amino acid sequences set forth in either SEQ ID NO:13 or SEQ ID NO:15.

Figure 11 is a model of a monomeric polypeptide that is representative of the monomeric subunits encoded by the amino acid sequences set forth in SEQ ID NO:14 and SEQ ID NO:16.

Figure 12 is a model of a dimeric polypeptide that is representative of the dimeric polypeptides formed from two monomeric polypeptides encoded by the amino acid sequences set forth in either SEQ ID NO:15 or SEQ ID NO:17.

Figure 13 is a model of a monomeric polypeptide that is representative of the monomeric subunit encoded by the amino acid sequence set forth in SEQ ID NO:18.

Figure 14 is a model of a dimeric polypeptide that is representative of the dimeric polypeptide formed from two monomeric polypeptides encoded by the amino acid sequence set forth in SEQ ID NO:19.

Figure 15 is a model of a monomeric polypeptide that is representative of the monomeric subunit encoded by the amino acid sequence set forth in SEQ ID NO:21.

Figure 16 is a model of a monomeric polypeptide that is representative of the monomeric subunit encoded by the amino acid sequence set forth in SEQ ID NO:22.

Figure 17 is a model of a monomeric polypeptide that is representative of the monomeric subunit encoded by the amino acid sequence set forth in SEQ ID NO:23.

Figure 18 is a model of a monomeric polypeptide that is representative of the monomeric subunits encoded by the amino acid sequences set forth in SEQ ID NO:24, SEQ ID NO:25, SEQ ID NO:26, and SEQ ID NO:27.

Figure 19 is a model of a monomeric polypeptide that is representative of the monomeric subunit encoded by the amino acid sequence set forth in SEQ ID NO:28.

Figure 20 is a model of a monomeric polypeptide that is representative of the monomeric subunit encoded by the amino acid sequence set forth in SEQ ID NO:29.

Figure 21 is a model of a monomeric polypeptide that is representative of the monomeric subunit encoded by the amino acid sequence set forth in SEQ ID NO:30.

Figure 22 is a model of a monomeric polypeptide that is representative of the monomeric subunit encoded by the amino acid sequence set forth in SEQ ID NO:31.

Figure 23 is a model of a monomeric polypeptide that is representative of the monomeric subunit encoded by the amino acid sequence set forth in SEQ ID NO:32.

Figure 24 is a model of a monomeric polypeptide that is representative of the monomeric subunit encoded by the amino acid sequence set forth in SEQ ID NO:33.

Figure 25 is a model of a monomeric polypeptide that is representative of the monomeric subunits encoded by the amino acid sequences set forth in SEQ ID NO:34, SEQ ID NO:35, SEQ ID NO:36, SEQ ID NO:37, and SEQ ID NO:38.

Figure 26 is a model of a monomeric polypeptide that is representative of the monomeric subunit encoded by the amino acid sequence set forth in SEQ ID NO 92.

Figure 27 is a model of a monomeric polypeptide that is representative of the monomeric subunit encoded by the amino acid sequence set forth in SEQ ID NO 93.

Figure 28 is a model of a monomeric polypeptide that is representative of the monomeric subunit encoded by the amino acid sequence set forth in SEQ ID NO 94.

Figure 29 is a model of a monomeric polypeptide that is representative of the monomeric subunit encoded by the amino acid sequence set forth in SEQ ID NO 95.

Figure 30 is a model of a monomeric polypeptide that is representative of the monomeric subunit encoded by the amino acid sequence set forth in SEQ ID NO 96.

Figure 31 is a model of a monomeric polypeptide that is representative of the monomeric subunit encoded by the amino acid sequence set forth in SEQ ID NO 97.

Figure 32 is a model of a monomeric polypeptide that is representative of the monomeric subunit encoded by the amino acid sequence set forth in SEQ ID NO 98.

Figure 33 is a model of a monomeric polypeptide that is representative of the monomeric subunit encoded by the amino acid sequence set forth in SEQ ID NO 99.

Figure 34 is a model of a monomeric polypeptide that is representative of the monomeric subunit encoded by the amino acid sequence set forth in SEQ ID NO 100.

Figure 35 is a model of a monomeric polypeptide that is representative of the monomeric subunit encoded by the amino acid sequence set forth in SEQ ID NO 101.

Figure 36 is a model of a monomeric polypeptide that is representative of the monomeric subunit encoded by the amino acid sequence set forth in SEQ ID NO 102.

Figure 37 is a model of a monomeric polypeptide that is representative of the monomeric subunit encoded by the amino acid sequence set forth in SEQ ID NO 103.

Figure 38 is a model of a monomeric polypeptide that is representative of the monomeric subunit encoded by the amino acid sequence set forth in SEQ ID NO 104.

Figure 39 is a model of a monomeric polypeptide that is representative of the monomeric subunit encoded by the amino acid sequence set forth in SEQ ID NO 105.

Figure 40 is a model of a monomeric polypeptide that is representative of the monomeric subunit encoded by the amino acid sequence set forth in SEQ ID NO 106.

Figure 44 is a model of a monomeric polypeptide that is representative of the monomeric subunit encoded by the amino acid sequence set forth in SEQ ID NO 91.

### Example 3. Cloning and expression of recombinant polypeptides

Polypeptides env_1, 2, 3, 4, 5, 8, 9, 10, 11, 12, 13, 14, 15, 16, 17, and env_18 were cloned, expressed in mammalian cells, and the cell supernatant was isolated. Protein from the cell supernatant was analyzed on 4-20% tris-glycine TGXTM sodium dodecyl sulfate polyacrylamide (SDS-PAGE) gels run under reducing and non-reducing conditions. The gels were stained with the InstantBlueTM protein stain. Polypeptides env_1, 2, 3, 5, 8, 9, 10, 11, 12, 13, 14, 15, 16, 17, and 18 expressed at detectable levels (Figures 41 and 42).

### Example 4. Mammalian expressed recombinant polypeptides are capable of detecting HIV antibodies in donor samples by ELISA

Enzyme immunosorbent assays (ELISA) were used to determine that mammalian expressed HIV env recombinant polypeptides are capable of detecting HIV antibodies in plasma samples from donors missed by the yeast expressed HIV antigen Env13 (control).

Briefly, the wells of a multi-well plate were coated overnight at room temperature with 50µl of each antigen at the same molar concentration including the control antigen Env13 (yeast). After incubation, the plates were washed twice with 340µl of wash buffer and then blocked with blocking buffer for 90 minutes at room temperature. After blocking the plates, the blocking buffer was aspirated out the wells and 5 µl of each donor sample was added to the wells together with 50µl of specimen diluent. The plates were then covered and incubated for 45 min at 37°C. After that, the plates were washed 4 times with 340µl of 1x wash buffer and 50µl of anti-IgG-HRP conjugate was added to each well. Plates were covered and incubated again for 45 min at 37°C and washed again with 340µl of 1x wash buffer. The substrate solution was prepared by dissolving a tablet of OPD (20mg) per 12ml of substrate buffer. Later, 50µl of substrate solution was added to each well and the plate was further incubated for 30min at room temperature at the dark. Finally, 15 µl of 4N H₂SO₄ was added to each well to stop the reaction. Bound antigens were quantified by monitoring the conversion of a horseradish peroxidase substrate into product by absortion spectroscopy at 490nm. A maximum of 337 samples were tested.

The control antigen Env13 (yeast) only detected positive hits in 307 samples out of 337 samples tested. Most of the mammalian derived env antigens tested were able to detect positive hits missed by Env13. All the samples missed by Env13(yeast) seemed to be low titer samples. However, mammalian derived env polypeptides were able to cover up to 4 samples missed by Env13 (yeast expressed). The results of Table 1 demonstrate that the new mammalian derived env recombinant polypeptides allow for detection of HIV antibodies in donor samples that were missed by the prior art antigen Env13 (yeast).

**Table 1. Detection of HIV antibodies in donor samples by HIV env recombinant polypeptides**

| **Env antigens** | **Total # Samples Tested** | **Total Positive Hits** | **# of unique hits (missed by Env13 (yeast))** |
|---|---|---|---|
| Control Env13 (yeast) | 337 | 307 | |
| env_5 | 337 | 224 | 4 |
| env_11 | 337 | 288 | 3 |
| env_20 | 337 | 280 | 4 |
| env_22 | 154 | 42 | 4 |
| env_23 | 146 | 109 | 3 |
| env_32 | 32 | 23 | 4 |
| env_40 | 31 | 25 | 4 |
| env_41 | 30 | 28 | 3 |
| env_43 | 154 | 112 | 4 |
| env_44 | 32 | 31 | 4 |
| env_46 | 136 | 101 | 4 |
| env_48 | 154 | 112 | 4 |

## Claims

1. A recombinant monomeric polypeptide, comprising
a) a leader peptide, and
b) a fusion protein, comprising
i) at least one epitope of the HIV gp120 protein, and
ii) at least one epitope of the HIV gp41 protein;
wherein the fusion protein comprises a region that is not encoded by HIV, said region consisting of a Fc domain of an antibody that lacks a hinge region,
wherein the recombinant polypeptide lacks a transmembrane domain, and
wherein the polypeptide include at least 4 epitopes of the HIV envelope protein.

2. The recombinant polypeptide according to claim 1, wherein said antibody Fc domain amino acid sequence is selected from the group consisting of SEQ ID. NO: 77 or SEQ ID. NO: 80.

3. The recombinant polypeptide of any one of the preceding claims, wherein the recombinant polypeptide further comprises a polyhistidine affinity tag.

4. The recombinant polypeptide of any of the preceding claims, wherein the leader peptide comprises the amino acid sequence set forth in SEQ ID NO:84.

5. An immunoassay reagent comprising a polypeptide according to any one of claims 1 to 4, wherein the immunoassay reagent is bound to a solid support.

6. A device for assaying a composition containing an HIV antibody, comprising a solid support and the polypeptide of any one of claims 1 to 4, wherein the polypeptide is covalently or non-covalently bound to the solid support.

7. The device of claim 6, wherein the solid support is a bead, a membrane, a microtiter plate, a polypeptide chip, or the solid-phase of a chromatography column.

8. A method of assaying a sample containing an antibody, comprising:
contacting the sample and the polypeptide of any one of claims 1 to 4; and
determining the binding affinity between the antibody and the polypeptide.

9. A method of selecting an anti-HIV antibody from a plurality of antibodies, comprising:
contacting
(a) the polypeptide of any one of claims 1 to 4 and
(b) a composition containing the plurality of antibodies; and
identifying an antibody that has a high binding affinity to the polypeptide relative to other antibodies in the composition, thereby selecting the anti-HIV antibody.

10. A recombinant polypeptide, according to any of claims 1 to 4, for use in the prevention of an HIV infection in a human patient, comprising administering to the patient the polypeptide of any one of claims 1 to 4.

## Patentansprüche

1. Rekombinantes monomeres Polypeptid, umfassend
a) ein Leader-Peptid, und
b) ein Fusionsprotein, umfassend
i) mindestens ein Epitop des HIV-gp120-Proteins, und
ii) mindestens ein Epitop des HIV-gp41-Proteins;
wobei das Fusionsprotein eine Region umfasst, die nicht von HIV kodiert wird, wobei die Region aus einer Fc-Domäne eines Antikörpers besteht, dem eine Gelenkregion fehlt,
wobei dem rekombinanten Polypeptid eine Transmembrandomäne fehlt, und
wobei das Polypeptid mindestens 4 Epitope des HIV-Hüllproteins beinhaltet.

2. Rekombinantes Polypeptid nach Anspruch 1, wobei die Aminosäuresequenz der Antikörper-Fc-Domäne ausgewählt ist aus der Gruppe, bestehend aus SEQ ID NO: 77 oder SEQ ID NO: 80.

3. Rekombinantes Polypeptid nach einem der vorhergehenden Ansprüche, wobei das rekombinante Polypeptid ferner einen Polyhistidin-Affinitäts-Tag umfasst.

4. Rekombinantes Polypeptid nach einem der vorhergehenden Ansprüche, wobei das Leader-Peptid die Aminosäuresequenz umfasst, die in SEQ ID NO: 84 dargelegt ist.

5. Immunassay-Reagenz, umfassend ein Polypeptid nach einem der Ansprüche 1 bis 4, wobei das Immunassay-Reagenz an einen festen Träger gebunden ist.

6. Vorrichtung zum Analysieren einer Zusammensetzung, die einen HIV-Antikörper enthält, umfassend einen festen Träger und das Polypeptid nach einem der Ansprüche 1 bis 4, wobei das Polypeptid kovalent oder nicht-kovalent an den festen Träger gebunden ist.

7. Vorrichtung nach Anspruch 6, wobei der feste Träger ein Kügelchen, eine Membran, eine Mikrotiterplatte, ein Polypeptidchip oder die feste Phase einer Chromatographiesäule ist.

8. Verfahren zum Analysieren einer Probe, die einen Antikörper enthält, umfassend:
Inkontaktbringen der Probe und des Polypeptids nach einem der Ansprüche 1 bis 4; und Bestimmen der Bindungsaffinität zwischen dem Antikörper und dem Polypeptid.

9. Verfahren zum Auswählen eines Anti-HIV-Antikörpers aus einer Vielzahl von Antikörpern, umfassend:
Inkontaktbringen
(a) des Polypeptids nach einem der Ansprüche 1 bis 4 und
(b) einer Zusammensetzung, die die Vielzahl von Antikörpern enthält, und
Identifizieren eines Antikörpers, der eine hohe Bindungsaffinität zu dem Polypeptid im Vergleich zu anderen Antikörpern in der Zusammensetzung aufweist, wodurch der Anti-HIV-Antikörper ausgewählt wird.

10. Rekombinantes Polypeptid nach einem der Ansprüche 1 bis 4 zur Verwendung bei der Prävention einer HIV-Infektion bei einem menschlichen Patienten, umfassend ein Verabreichen des Polypeptids nach einem der Ansprüche 1 bis 4 an den Patienten.

## Revendications

1. Polypeptide monomère recombinant, comprenant
a) un peptide leader, et
b) une protéine de fusion, comprenant
i) au moins un épitope de la protéine gp120 du VIH, et
ii) au moins un épitope de la protéine gp41 du VIH ;
dans lequel la protéine de fusion comprend une région qui n'est pas codée par le VIH, ladite région étant constituée d'un domaine Fc d'un anticorps dépourvu de région charnière,
dans lequel le polypeptide recombinant est dépourvu de domaine transmembranaire, et
dans lequel le polypeptide comprend au moins 4 épitopes de la protéine d'enveloppe du VIH.

2. Polypeptide recombinant selon la revendication 1, dans lequel ladite séquence d'acides aminés du domaine Fc de l'anticorps est sélectionnée dans le groupe constitué de SEQ ID n° : 77, ou SEQ ID n° : 80.

3. Polypeptide recombinant selon l'une quelconque des revendications précédentes, dans lequel le polypeptide recombinant comprend en outre un marqueur d'affinité polyhistidine.

4. Polypeptide recombinant selon l'une quelconque des revendications précédentes, dans lequel le peptide leader comprend la séquence d'acides aminés décrite dans SEQ ID n° : 84.

5. Réactif d'immunoanalyse comprenant un polypeptide selon l'une quelconque des revendications 1 à 4, dans lequel le réactif d'immunoanalyse est lié à un support solide.

6. Dispositif d'analyse d'une composition contenant un anticorps anti-VIH, comprenant un support solide et le polypeptide selon l'une quelconque des revendications 1 à 4, dans lequel le polypeptide est lié de manière covalente ou non covalente au support solide.

7. Dispositif selon la revendication 6, dans lequel le support solide est une bille, une membrane, une plaque de microtitrage, une puce polypeptidique ou la phase solide d'une colonne de chromatographie.

8. Procédé d'analyse d'un échantillon contenant un anticorps, comprenant :
la mise en contact de l'échantillon et du polypeptide selon l'une quelconque des revendications 1 à 4 ; et
la détermination de l'affinité de liaison entre l'anticorps et le polypeptide.

9. Procédé de sélection d'un anticorps anti-VIH parmi une pluralité d'anticorps, comprenant :
la mise en contact
(a) du polypeptide selon l'une quelconque des revendications 1 à 4 et
(b) d'une composition contenant la pluralité d'anticorps ; et
l'identification d'un anticorps qui a une affinité de liaison élevée pour le polypeptide par rapport à d'autres anticorps dans la composition, sélectionnant ainsi l'anticorps anti-VIH.

10. Polypeptide recombinant selon l'une quelconque des revendications 1 à 4, destiné à être utilisé dans la prévention d'une infection par le VIH chez un patient humain, comprenant l'administration au patient du polypeptide selon l'une quelconque des revendications 1 à 4.
